(19) Europäisches Patentamt
European Patent Office
Office européen des brevets

(11) EP 4 234 537 A1

(12) EUROPEAN PATENT APPLICATION
published in accordance with Art. 153(4) EPC

(43) Date of publication:
**30.08.2023 Bulletin 2023/35**

(21) Application number: **21882902.6**

(22) Date of filing: **21.10.2021**

(51) International Patent Classification (IPC):
***C07C 321/04*** *(2006.01)* ***C07C 321/26*** *(2006.01)*
***C07D 333/38*** *(2006.01)* ***G01N 31/00*** *(2006.01)*
***C07D 213/72*** *(2006.01)* ***G01N 33/15*** *(2006.01)*
***G01N 21/17*** *(2006.01)* ***G01N 21/31*** *(2006.01)*
***G01N 21/64*** *(2006.01)* ***G01N 30/06*** *(2006.01)*

(52) Cooperative Patent Classification (CPC):
**C07C 323/60; C07C 323/41; G01N 30/06; G01N 31/00; G01N 33/15;** C07C 2601/02

(86) International application number:
**PCT/JP2021/038929**

(87) International publication number:
**WO 2022/085762 (28.04.2022 Gazette 2022/17)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **22.10.2020 JP 2020177157**

(71) Applicant: **FUJIFILM Corporation**
**Tokyo 106-8620 (JP)**

(72) Inventors:
- **TAMURA Takashi**
  **Ashigarakami-gun, Kanagawa 258-8577 (JP)**
- **FUJITA Masaharu**
  **Ashigarakami-gun, Kanagawa 258-8577 (JP)**
- **YAMAMOTO Yusuke**
  **Ashigarakami-gun, Kanagawa 258-8577 (JP)**
- **TAKAKU Koji**
  **Ashigarakami-gun, Kanagawa 258-8577 (JP)**

(74) Representative: **HGF**
**HGF Limited**
**1 City Walk**
**Leeds LS11 9DX (GB)**

# (54) REAGENT FOR MEASURING SKIN SENSITIZATION, COMPOUND, AND METHOD FOR MEASURING SKIN SENSITIZATION

(57) An object of the present invention is to provide a reagent for measuring skin sensitization that can measure sensitization to a test substance with high sensitivity using a single type of reagent; a compound; and a method for measuring skin sensitization. According to the present invention, provided are a reagent for measuring skin sensitization including, as a main measuring agent, an organic compound having a mercapto group and a hydrazide structure and having an absorption spectrum in an ultraviolet, visible, or near-infrared region; a compound for use in the reagent for measuring skin sensitization; and a method for measuring skin sensitization using the reagent for measuring skin sensitization.

EP 4 234 537 A1

## Description

Field of the Invention

The present invention relates to a reagent for measuring skin sensitization, a compound, and a method for measuring skin sensitization.

Backgroud Art

Skin sensitization (allergy) is not limited to symptoms such as localized blisters and erythema at a site of exposure to a certain substance, and may be accompanied by a serious and life-threatening systemic allergic reaction called anaphylaxis. In addition, skin sensitization is considered to be one of the significant toxicities because once the skin sensitization develops, management to avoid long-term exposure to the allergic substance is required.

Conventionally, a test method using a guinea pig has been generally known as a method for evaluating the skin sensitization of a chemical substance, and a test method such as a guinea pig maximisation test (GPMT) using an adjuvant or a Buehler Test which is a non-adjuvant test has been widely used for many years. On the other hand, in recent years, research and development of alternatives to animal experiments have been promoted due to ethical and social demands such as animal welfare.

An in vitro test is mainly being developed as a skin sensitization test method that does not use an animal. ARE-Nrf2 luciferase KeratinoSens™ test method (KeratinoSens is a registered trademark), LuSens (ARE-Nrf2 luciferase LuSens test method), h-CLAT (human Cell Line Activation Test), U-SENS (Myeloid U937 Skin Sensitization Test), IL-8 Luc assay, and the like are known as the in vitro test.

On the other hand, there is an in chemico test as a test method that does not use cultured cells. The in chemico test based on a chemical reaction does not use cultured cells, and therefore has many advantages such as no need for special techniques, knowledge, and equipment. For example, [Gerberick, G. F., Vassallo, J. D., Bailey, R. E., Chaney, J. G., Morrall, S. W., and Lepoittevin, J. P. (2004). Development of a peptide reactivity assay for screening contact allergens. Toxicological Sciences, 81 (2), pp. 332-343] and [Gerberick, G. F., Vassallo, J. D., Foertsch, L. M., Price, B. B., Chaney, J. G., and Lepoittevin, J. P. (2007). Quantification of chemical peptide reactivity for screening contact allergens: a classification tree model approach. Toxicological Sciences, 97 (2), pp. 417-427] describe a method using two types of peptides (a cysteine peptide and a lysine peptide) as nucleophilic reagents. In addition, JP2011-59102A and JP2014-37995A describe a reagent for measuring skin sensitization and a method for measuring skin sensitization, using a cysteine derivative into which an aryl ring has been introduced and a lysine derivative into which an aryl ring has been introduced as nucleophilic reagents (also referred to as ADRA).

In the methods described in Non-Patent Documents 1 and 2 and Patent Documents 1 and 2, two types of reagents containing cysteine and lysine are separately chemically reacted with a test substance, and % depletion of cysteine and lysine is calculated by separately measuring and quantifying cysteine and lysine, so the evaluation takes time. Therefore, test methods for detecting and evaluating a skin sensitizing substance using a peptide containing these two types of amino acids have also been reported. However, all of the test methods using a peptide containing two amino acid types of cysteine and lysine are test methods in which a synthetic heptapeptide Cor1C-420 (Ac-Asn-Lys-Lys-Cys-Asp-Leu-Phe) (derived from the sequence around cysteine at the 420th residue from the N-terminal of the human Coronin 1 protein, which is a site that exhibits an extremely high reactivity with an electrophilic reagent, [Non-Patent Document 3] is used and the measurement is carried out by liquid chromatography/mass spectrometry (LC-MS), all of which have low detection sensitivity and are therefore test methods in which optical detection of UV, visible light, or the like is impossible. In addition, shortening of the measurement time by including cysteine and lysine is not regarded as an effect.

Non-Patent Document 4 describes five points: (1) peptide-test substance adducts (covalent conjugates) can be distinguished from oxidation of peptides, (2) there is no problem of precipitation of the test substance since the concentration of the test substance in a reaction solution can be reduced, (3) the problem of solubility of the test substance is reduced since the preparation concentration of the test substance can be reduced, (4) there is no problem of co-elution since the measurement is carried out by LC-MS, and (5) carrying out kinetic measurement makes it possible to obtain more accurate evaluation of highly reactive test substances. In addition, Non-Patent Document 5 describes that high prediction accuracy can be obtained by evaluating three types of peptides: the cysteine peptide and the lysine peptide used in the direct peptide reactivity assay (DPRA) described in Non-Patent Document 1 and 2, and the synthetic heptapeptide Cor1C-420 described in Non-Patent Document 3. Further, Patent Document 3 describes a reagent for detecting skin sensitization in which a fluorescent dye is bonded to a terminal of a peptide having an amino group and a thiol group in the same molecule.

Prior Art Documents

Non-Patent Documents

Non-Patent Document 1: Gerberick, G. F., Vassallo, J. D., Bailey, R. E., Chaney, J. G., Morrall, S. W., and Lepoittevin, J. P. (2004). Development of a peptide reactivity assay for screening contact allergens. Toxicological Sciences, 81 (2), pp. 332-343

Non-Patent Document 2: Gerberick, G. F., Vassallo, J. D., Foertsch, L. M., Price, B. B., Chaney, J. G., and Lepoittevin, J. P. (2007). Quantification of chemical peptide reactivity for screening contact allergens: a classification tree model approach. Toxicological Sciences, 97 (2), pp. 417-427

Non-Patent Document 3: Dennehy M. K., Richards K. A. M., Wernke G. R., Shyr Y, and Liebler D. C. (2006). Cytosolic and nuclear protein targets of thiol-reactive electrophiles. Chemical Research in Toxicology, 19, pp. 20-29

Non-Patent Document 4: Natsch A. and Gfeller H. (2008). LC-MS-based characterization of the peptide reactivity of chemicals to improve the in vitro prediction of the skin sensitization potential. Toxicological Sciences, 106 (2), pp. 464-478

Non-Patent Document 5: Wong C. L., Lam A. L., Smith M. T., Ghassabian S. (2016). Evaluation of a High-Throughput Peptide Reactivity Format Assay for Assessment of the Skin Sensitization Potential of Chemicals. Frontiers in Pharmacology, 14, 7 (53), pp. 1-14

Patent Documents

Patent Document 1: JP2011-59102A
Patent Document 2: JP2014-37995A
Patent Document 3: JP2009-222466A

## SUMMARY OF THE INVENTION

In the method for measuring skin sensitization described in [Gerberick, G. F., Vassallo, J. D., Bailey, R. E., Chaney, J. G., Morrall, S. W., and Lepoittevin, J. P. (2004). Development of a peptide reactivity assay for screening contact allergens. Toxicological Sciences, 81 (2), pp. 332-343] and [Gerberick, G. F., Vassallo, J. D., Foertsch, L. M., Price, B. B., Chaney, J. G., and Lepoittevin, J. P. (2007). Quantification of chemical peptide reactivity for screening contact allergens: a classification tree model approach. Toxicological Sciences, 97 (2), pp. 417-427], the two types of peptides used have low molar absorption coefficients and can only be detected at a short wavelength of 220 nm, so quantitation of the residual ratio of these peptides by the HPLC-UV method has problems such as low quantitative sensitivity and frequent occurrence of phenomena such as co-elution of peptides and test substances, making quantification difficult in many cases. The method described in [Dennehy M. K., Richards K. A. M., Wernke G. R., Shyr Y, and Liebler D. C. (2006). Cytosolic and nuclear protein targets of thiol-reactive electrophiles. Chemical Research in Toxicology, 19, pp. 20-29] is a test method in which optical detection of UV, visible light, or the like is difficult, so there is a problem that the evaluation by mass spectrometry is necessary. In addition, this test method does not claim to achieve an effect of shortening the measurement time by including cysteine and lysine. [Natsch A. and Gfeller H. (2008). LC-MS-based characterization of the peptide reactivity of chemicals to improve the in vitro prediction of the skin sensitization potential. Toxicological Sciences, 106 (2), pp. 464-478] does not describe efficiency improvement by shortening the measurement time. The method described in [Wong C. L., Lam A. L., Smith M. T., Ghassabian S. (2016). Evaluation of a High-Throughput Peptide Reactivity Format Assay for Assessment of the Skin Sensitization Potential of Chemicals. Frontiers in Pharmacology, 14, 7 (53), pp. 1-14] has a problem that the optical quantitative sensitivity is low. In addition, the peptide used in the method described in JP2009-222466A has a thiol group derived from cysteine and an $\alpha$-amino group of an amino acid, but this peptide has a low reactivity with a test substance having weak skin sensitization, sometimes leading to a case where it is determined as a false negative, which has been a problem.

An object of the present invention is to provide a reagent for measuring skin sensitization that can measure sensitization to a test substance with high sensitivity using a single type of reagent; a compound; and a method for measuring skin sensitization.

As a result of extensive studies to achieve the above object, the present inventors have found that an organic compound having a mercapto group and a hydrazide structure and having an absorption spectrum in an ultraviolet, visible, or near-infrared region can be used as a reagent for measuring skin sensitization. The present invention has

been completed based on these findings. According to the present invention, the following inventions are provided.

<1> A reagent for measuring skin sensitization comprising, as a main measuring agent, an organic compound having a mercapto group and a hydrazide structure and having an absorption spectrum in an ultraviolet, visible, or near-infrared region.

<2> The reagent for measuring skin sensitization according to <1>, in which the organic compound is represented by Formula (1) or Formula (2).

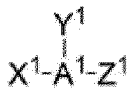 (1)

$X^2$-$Y^2$-$Z^2$ (2)

In the formulae,

$A^1$ represents a nitrogen atom or the following linking group.

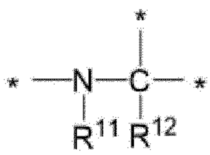

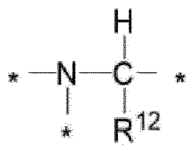

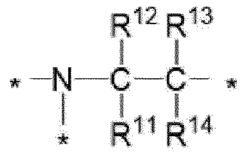

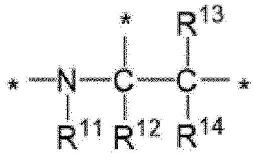

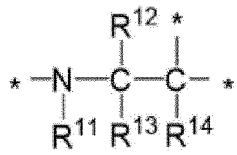

$R^{11}$, $R^{12}$, $R^{13}$, and $R^{14}$ each independently represent a hydrogen atom, an alkyl group having 1 to 10 carbon atoms, an alkenyl group having 2 to 10 carbon atoms, an alkynyl group having 2 to 10 carbon atoms, a cycloalkyl group having 3 to 10 carbon atoms, or a cycloalkenyl group having 3 to 10 carbon atoms, each of which may contain -O-, -C(O)-, -OC(O)-, -$NJ^1$-CO-, -CO-$NJ^1$-, or -NH-CO-NH- in a molecular chain, $J^1$ represents a hydrogen atom or an alkyl group having 1 to 3 carbon atoms, and the alkyl group, the alkenyl group, the alkynyl group, the cycloalkyl group, or the cycloalkenyl group may have a substituent selected from a cycloalkyl group having 3 to 6 carbon atoms, a cycloalkenyl group having 5 to 6 carbon atoms, an amino group, a cyano group, a mercapto group, a mercaptomethyl group, a hydroxyl group, a phenyl group, a hydroxyphenyl group, a pyridyl group, a naphthyl group, a thienyl group, or a furyl group.

* represents a connection position with $X^1$, $Y^1$, or $Z^1$.

$X^1$ and $X^2$ represent an alkyl group having one or more mercapto groups and having 1 to 10 carbon atoms, an alkenyl group having one or more mercapto groups and having 2 to 10 carbon atoms, an alkynyl group having one or more mercapto groups and having 2 to 10 carbon atoms, a cycloalkyl group having one or more mercapto groups and having 3 to 10 carbon atoms, a cycloalkenyl group having one or more mercapto groups and having 3 to 10 carbon atoms, an arylalkyl group having one or more mercapto groups and having 7 to 12 carbon atoms, a heteroalkylalkyl group having one or more mercapto groups and having 3 to 10 carbon atoms, or a mercapto group, each of which may contain -O-, -C(O)-, -OC(O)-, -$NJ^2$-CO-, -CO-$NJ^2$-, or -NH-CO-NH- in a molecular chain, $J^2$ represents a hydrogen atom or an alkyl group having 1 to 3 carbon atoms, and the alkyl group, the alkenyl group, the alkynyl group, the cycloalkyl group, or the cycloalkenyl group may have a substituent selected from a cycloalkyl group having 3 to 6 carbon atoms, a cycloalkenyl group having 5 to 6 carbon atoms, an amino group, a cyano group, a mercaptomethyl group, a hydroxyl group, a phenyl group, a hydroxyphenyl group, a pyridyl group, a naphthyl group, a thienyl group, or a furyl group.

$Y^1$ and $Y^2$ represent a group having 6 to 20 carbon atoms and containing a structure having an absorption spectrum in an ultraviolet, visible, or near-infrared region.

$Z^1$ and $Z^2$ represent -CO-$NR^{21}NR^{22}R^{23}$, where $R^{21}$, $R^{22}$, and $R^{23}$ each independently represent a hydrogen atom or an alkyl group having 1 to 10 carbon atoms.

<3> The reagent for measuring skin sensitization according to <1>, in which the organic compound is represented

by Formula (10).

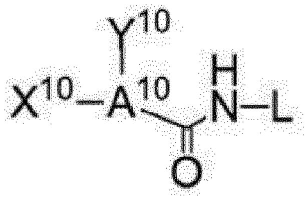

(10)

In the formula,

$A^{10}$ represents a nitrogen atom or a trivalent linking group, and
$X^{10}$ represents an alkyl group having one or more mercapto groups and having 1 to 10 carbon atoms, an alkenyl group having one or more mercapto groups and having 2 to 10 carbon atoms, an alkynyl group having one or more mercapto groups and having 2 to 10 carbon atoms, a cycloalkyl group having one or more mercapto groups and having 3 to 10 carbon atoms, a cycloalkenyl group having one or more mercapto groups and having 3 to 10 carbon atoms, an arylalkyl group having one or more mercapto groups and having 7 to 12 carbon atoms, a heteroalkylalkyl group having one or more mercapto groups and having 3 to 10 carbon atoms, or a mercapto group, each of which may contain -O-, -C(O)-, -OC(O)-, $-NJ^{101}$-CO-, $-CO-NJ^{101}$-, or -NH-CO-NH- in a molecular chain, $J^{101}$ represents a hydrogen atom or an alkyl group having 1 to 3 carbon atoms, and the alkyl group, the alkenyl group, the alkynyl group, the cycloalkyl group, or the cycloalkenyl group may have a substituent selected from a cycloalkyl group having 3 to 6 carbon atoms, a cycloalkenyl group having 5 to 6 carbon atoms, an amino group, a cyano group, a mercaptomethyl group, a hydroxyl group, a phenyl group, a hydroxyphenyl group, a pyridyl group, a naphthyl group, a thienyl group, or a furyl group.
$Y^{10}$ represents a group having 6 to 20 carbon atoms and containing a structure having an absorption spectrum in an ultraviolet, visible, or near-infrared region, and
L represents an amino group.

<4> The reagent for measuring skin sensitization according to <1>, in which the organic compound is represented by Formula (3), Formula (4), or Formula (5).

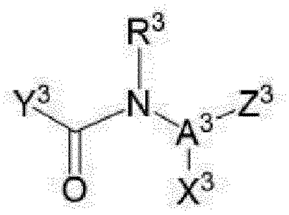

(3)

In the formula,

$A^3$ represents a trivalent hydrocarbon group having 1 or 2 carbon atoms, and
$R^3$ represents a hydrogen atom, an alkyl group having 1 to 10 carbon atoms, or a cycloalkyl group having 3 to 10 carbon atoms, each of which may contain -O-, -C(O)-, -OC(O)-, $-NJ^{31}$-CO-, $-CO-NJ^{31}$-, or -NH-CO-NH- in a molecular chain, $J^{31}$ represents a hydrogen atom or an alkyl group having 1 to 3 carbon atoms, and the alkyl group or the cycloalkyl group may have a substituent selected from a cycloalkyl group having 3 to 6 carbon atoms, an amino group, a cyano group, a mercapto group, a hydroxyl group, a carboxyl group, a phenyl group, a hydroxyphenyl group, a pyridyl group, a naphthyl group, a thienyl group, or a furyl group.
$X^3$ represents an alkyl group having one or more mercapto groups and having 1 to 10 carbon atoms, a cycloalkyl group having one or more mercapto groups and having 3 to 10 carbon atoms, an arylalkyl group having one or more mercapto groups and having 7 to 12 carbon atoms, a heteroalkylalkyl group having one or more mercapto groups and having 3 to 10 carbon atoms, or a mercapto group, each of which may contain -O-, -C(O)-, -OC(O)-, $-NJ^{32}$-CO-, $-CO-NJ^{32}$-, or -NH-CO-NH- in a molecular chain, $J^{32}$ represents a hydrogen atom or an alkyl group having 1 to 3 carbon atoms, and the alkyl group or the cycloalkyl group may have a substituent selected from a cycloalkyl group having 3 to 6 carbon atoms, an amino group, a cyano group, a hydroxyl group, a carboxyl group, a phenyl group, a hydroxyphenyl group, a pyridyl group, a naphthyl group, a thienyl group, or a furyl group.
$Y^3$ represents a group having 6 to 20 carbon atoms and containing a structure having an absorption spectrum

in an ultraviolet, visible, or near-infrared region.
$Z^3$ represents $-CO-NR^{31}NR^{32}R^{33}$, where $R^{31}$, $R^{32}$, and $R^{33}$ each independently represent a hydrogen atom or an alkyl group having 1 to 10 carbon atoms.

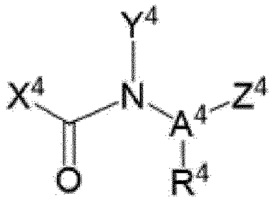
(4)

In the formula,

$A^4$ represents a trivalent hydrocarbon group having 1 or 2 carbon atoms, and
$R^4$ represents a hydrogen atom, an alkyl group having 1 to 10 carbon atoms, or a cycloalkyl group having 3 to 10 carbon atoms, each of which may contain -O-, -C(O)-, -OC(O)-, $-NJ^{41}-CO-$, $-CO-NJ^{41}-$, or -NH-CO-NH- in a molecular chain, $J^{41}$ represents a hydrogen atom or an alkyl group having 1 to 3 carbon atoms, and the alkyl group or the cycloalkyl group may have a substituent selected from a cycloalkyl group having 3 to 6 carbon atoms, an amino group, a cyano group, a mercapto group, a hydroxyl group, a carboxyl group, a phenyl group, a hydroxyphenyl group, a pyridyl group, a naphthyl group, a thienyl group, or a furyl group.
$X^4$ represents an alkyl group having one or more mercapto groups and having 1 to 10 carbon atoms, a cycloalkyl group having one or more mercapto groups and having 3 to 10 carbon atoms, an arylalkyl group having one or more mercapto groups and having 7 to 12 carbon atoms, a heteroalkylalkyl group having one or more mercapto groups and having 3 to 10 carbon atoms, or a mercapto group, each of which may contain -O-, -C(O)-, -OC(O)-, $-NJ^{42}-CO-$, $-CO-NJ^{42}-$, or -NH-CO-NH- in a molecular chain, $J^{42}$ represents a hydrogen atom or an alkyl group having 1 to 3 carbon atoms, and the alkyl group or the cycloalkyl group may have a substituent selected from a cycloalkyl group having 3 to 6 carbon atoms, an amino group, a cyano group, a hydroxyl group, a carboxyl group, a phenyl group, a hydroxyphenyl group, a pyridyl group, a naphthyl group, a thienyl group, or a furyl group.
$Y^4$ represents a group having 6 to 20 carbon atoms and containing a structure having an absorption spectrum in an ultraviolet, visible, or near-infrared region.
$Z^4$ represents $-CO-NR^{41}NR^{42}R^{43}$, where $R^{41}$, $R^{42}$, and $R^{43}$ each independently represent a hydrogen atom or an alkyl group having 1 to 10 carbon atoms.

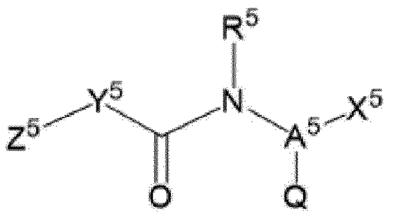
(5)

In the formula,

$A^5$ represents a trivalent hydrocarbon group having 1 or 2 carbon atoms, and
$R^5$ represents a hydrogen atom, an alkyl group having 1 to 10 carbon atoms, or a cycloalkyl group having 3 to 10 carbon atoms, each of which may contain -O-, -C(O)-, -OC(O)-, $-NJ^{51}-CO-$, $-CO-NJ^{51}-$, or -NH-CO-NH- in a molecular chain, $J^{51}$ represents a hydrogen atom or an alkyl group having 1 to 3 carbon atoms, and the alkyl group or the cycloalkyl group may have a substituent selected from a cycloalkyl group having 3 to 6 carbon atoms, an amino group, a cyano group, a mercapto group, a hydroxyl group, a carboxyl group, a phenyl group, a hydroxyphenyl group, a pyridyl group, a naphthyl group, a thienyl group, or a furyl group.
$X^5$ represents an alkyl group having one or more mercapto groups and having 1 to 10 carbon atoms, a cycloalkyl group having one or more mercapto groups and having 3 to 10 carbon atoms, an arylalkyl group having one or more mercapto groups and having 7 to 12 carbon atoms, a heteroalkylalkyl group having one or more mercapto groups and having 3 to 10 carbon atoms, or a mercapto group, each of which may contain -O-, -C(O)-, -OC(O)-, $-NT^{52}-CO-$, $-CO-NJ^{52}-$, or -NH-CO-NH- in a molecular chain, $J^{52}$ represents a hydrogen atom or an alkyl group having 1 to 3 carbon atoms, and the alkyl group or the cycloalkyl group may have a substituent selected from a cycloalkyl group having 3 to 6 carbon atoms, an amino group, a cyano group, a hydroxyl group, a carboxyl

group, a phenyl group, a hydroxyphenyl group, a pyridyl group, a naphthyl group, a thienyl group, or a furyl group.
$Y^5$ represents a group having 6 to 20 carbon atoms and containing a structure having an absorption spectrum in an ultraviolet, visible, or near-infrared region.
$Z^5$ represents -CO-$NR^{51}NR^{52}R^{53}$, where $R^{51}$, $R^{52}$, and $R^{53}$ each independently represent a hydrogen atom or an alkyl group having 1 to 10 carbon atoms.
Q represents a hydrogen atom, a carboxyl group, a hydroxyl group, or a primary amide structure, or represents -CO-$NR^{5a}NR^{5b}R^{5c}$, where $R^{5a}$, $R^{5b}$, and $R^{5c}$ each independently represent a hydrogen atom or an alkyl group having 1 to 10 carbon atoms.

<5> The reagent for measuring skin sensitization according to <1>, in which the organic compound is represented by Formula (6).

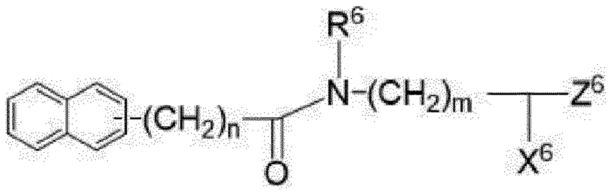
(6)

In the formula,

$R^6$ represents a hydrogen atom, an alkyl group having 1 to 10 carbon atoms, or a cycloalkyl group having 3 to 10 carbon atoms, each of which may contain -O-, -C(O)-, -OC(O)-, -$NJ^{61}$-CO-, -CO-$NJ^{61}$-, or -NH-CO-NH- in a molecular chain, $J^{61}$ represents a hydrogen atom or an alkyl group having 1 to 3 carbon atoms, and the alkyl group or the cycloalkyl group may have a substituent selected from a cycloalkyl group having 3 to 6 carbon atoms, an amino group, a cyano group, a mercapto group, a hydroxyl group, a carboxyl group, a phenyl group, a hydroxyphenyl group, a pyridyl group, a naphthyl group, a thienyl group, or a furyl group.
$X^6$ represents an alkyl group having one or more mercapto groups and having 1 to 10 carbon atoms, a cycloalkyl group having one or more mercapto groups and having 3 to 10 carbon atoms, an arylalkyl group having one or more mercapto groups and having 7 to 12 carbon atoms, a heteroalkylalkyl group having one or more mercapto groups and having 3 to 10 carbon atoms, or a mercapto group, each of which may contain -O-, -C(O)-, -OC(O)-, -$NJ^{62}$-CO-, -CO-$NJ^{62}$-, or -NH-CO-NH- in a molecular chain, $J^{62}$ represents a hydrogen atom or an alkyl group having 1 to 3 carbon atoms, and the alkyl group or the cycloalkyl group may have a substituent selected from a cycloalkyl group having 3 to 6 carbon atoms, an amino group, a cyano group, a hydroxyl group, a carboxyl group, a phenyl group, a hydroxyphenyl group, a pyridyl group, a naphthyl group, a thienyl group, or a furyl group.
$Z^6$ represents -CO-$NR^{61}NR^{62}R^{63}$, where $R^{61}$, $R^{62}$, and $R^{63}$ each independently represent a hydrogen atom or an alkyl group having 1 to 10 carbon atoms.
n represents 0 or 1, and
m represents 0 or 1.

<6> The reagent for measuring skin sensitization according to <1>, in which the organic compound is represented by Formula (7).

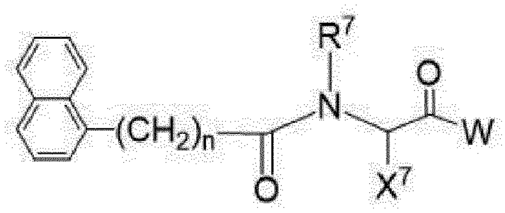
(7)

In the formula,

$R^7$ represents a hydrogen atom, an alkyl group having 1 to 10 carbon atoms, or a cycloalkyl group having 3 to 10 carbon atoms, each of which may contain -O-, -C(O)-, -OC(O)-, -$NJ^{71}$-CO-, -CO-$NJ^{71}$-, or -NH-CO-NH- in a molecular chain, $J^{71}$ represents a hydrogen atom or an alkyl group having 1 to 3 carbon atoms, and the alkyl group or the cycloalkyl group may have a substituent selected from a cycloalkyl group having 3 to 6 carbon

atoms, an amino group, a cyano group, a mercapto group, a hydroxyl group, a carboxyl group, a phenyl group, a hydroxyphenyl group, a pyridyl group, a naphthyl group, a thienyl group, or a furyl group.

$X^7$ represents an alkyl group having one or more mercapto groups and having 1 to 10 carbon atoms, a cycloalkyl group having one or more mercapto groups and having 3 to 10 carbon atoms, an arylalkyl group having one or more mercapto groups and having 7 to 12 carbon atoms, a heteroalkylalkyl group having one or more mercapto groups and having 3 to 10 carbon atoms, or a mercapto group, each of which may contain -O-, -C(O)-, -OC(O)-, $-NJ^{72}$-CO-, -CO-$NJ^{72}$-, or -NH-CO-NH- in a molecular chain, $J^{72}$ represents a hydrogen atom or an alkyl group having 1 to 3 carbon atoms, and the alkyl group or the cycloalkyl group may have a substituent selected from a cycloalkyl group having 3 to 6 carbon atoms, an amino group, a cyano group, a hydroxyl group, a carboxyl group, a phenyl group, a hydroxyphenyl group, a pyridyl group, a naphthyl group, a thienyl group, or a furyl group.

W represents $NR^{71}$-$NR^{72}R^{73}$, where $R^{71}$, $R^{72}$, and $R^{73}$ represent a hydrogen atom or an alkyl group having 1 to 3 carbon atoms, and

n represents 0 or 1.

<7> The reagent for measuring skin sensitization according to any one of <2> to <4>, in which $Y^1$, $Y^2$, $Y^3$, $Y^4$, $Y^5$, and $Y^{10}$ are groups that emit fluorescence.

<8> A compound represented by Formula (3), Formula (4), or Formula (5).

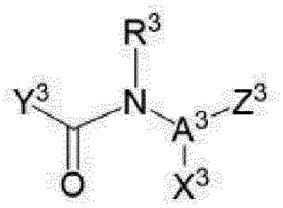

(3)

In the formula,

$A^3$ represents a trivalent hydrocarbon group having 1 or 2 carbon atoms, and

$R^3$ represents a hydrogen atom, an alkyl group having 1 to 10 carbon atoms, or a cycloalkyl group having 3 to 10 carbon atoms, each of which may contain -O-, -C(O)-, -OC(O)-, $-NJ^{31}$-CO-, -CO-$NJ^{31}$-, or -NH-CO-NH- in a molecular chain, $J^{31}$ represents a hydrogen atom or an alkyl group having 1 to 3 carbon atoms, and the alkyl group or the cycloalkyl group may have a substituent selected from a cycloalkyl group having 3 to 6 carbon atoms, an amino group, a cyano group, a mercapto group, a hydroxyl group, a carboxyl group, a phenyl group, a hydroxyphenyl group, a pyridyl group, a naphthyl group, a thienyl group, or a furyl group.

$X^3$ represents an alkyl group having one or more mercapto groups and having 1 to 10 carbon atoms, a cycloalkyl group having one or more mercapto groups and having 3 to 10 carbon atoms, an arylalkyl group having one or more mercapto groups and having 7 to 12 carbon atoms, a heteroalkylalkyl group having one or more mercapto groups and having 3 to 10 carbon atoms, or a mercapto group, each of which may contain -O-, -C(O)-, -OC(O)-, $-NJ^{32}$-CO-, -CO-$NJ^{32}$-, or -NH-CO-NH- in a molecular chain, $J^{32}$ represents a hydrogen atom or an alkyl group having 1 to 3 carbon atoms, and the alkyl group or the cycloalkyl group may have a substituent selected from a cycloalkyl group having 3 to 6 carbon atoms, an amino group, a cyano group, a hydroxyl group, a carboxyl group, a phenyl group, a hydroxyphenyl group, a pyridyl group, a naphthyl group, a thienyl group, or a furyl group.

$Y^3$ represents a group having 6 to 20 carbon atoms and containing a structure having an absorption spectrum in an ultraviolet, visible, or near-infrared region.

$Z^3$ represents -CO-$NR^{31}NR^{32}R^{33}$, where $R^{31}$, $R^{32}$, and $R^{33}$ each independently represent a hydrogen atom or an alkyl group having 1 to 10 carbon atoms.

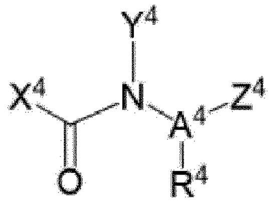

(4)

In the formula,

$A^4$ represents a trivalent hydrocarbon group having 1 or 2 carbon atoms, and
$R^4$ represents a hydrogen atom, an alkyl group having 1 to 10 carbon atoms, or a cycloalkyl group having 3 to 10 carbon atoms, each of which may contain -O-, -C(O)-, -OC(O)-, $-NJ^{41}-CO-$, $-CO-NJ^{41}-$, or -NH-CO-NH- in a molecular chain, $J^{41}$ represents a hydrogen atom or an alkyl group having 1 to 3 carbon atoms, and the alkyl group or the cycloalkyl group may have a substituent selected from a cycloalkyl group having 3 to 6 carbon atoms, an amino group, a cyano group, a mercapto group, a hydroxyl group, a carboxyl group, a phenyl group, a hydroxyphenyl group, a pyridyl group, a naphthyl group, a thienyl group, or a furyl group.
$X^4$ represents an alkyl group having one or more mercapto groups and having 1 to 10 carbon atoms, a cycloalkyl group having one or more mercapto groups and having 3 to 10 carbon atoms, an arylalkyl group having one or more mercapto groups and having 7 to 12 carbon atoms, a heteroalkylalkyl group having one or more mercapto groups and having 3 to 10 carbon atoms, or a mercapto group, each of which may contain -O-, -C(O)-, -OC(O)-, $-NJ^{42}-CO-$, $-CO-NJ^{42}-$, or -NH-CO-NH- in a molecular chain, $J^{42}$ represents a hydrogen atom or an alkyl group having 1 to 3 carbon atoms, and the alkyl group or the cycloalkyl group may have a substituent selected from a cycloalkyl group having 3 to 6 carbon atoms, an amino group, a cyano group, a hydroxyl group, a carboxyl group, a phenyl group, a hydroxyphenyl group, a pyridyl group, a naphthyl group, a thienyl group, or a furyl group.
$Y^4$ represents a group having 6 to 20 carbon atoms and containing a structure having an absorption spectrum in an ultraviolet, visible, or near-infrared region.
$Z^4$ represents $-CO-NR^{41}NR^{42}R^{43}$, where $R^{41}$, $R^{42}$, and $R^{43}$ each independently represent a hydrogen atom or an alkyl group having 1 to 10 carbon atoms.

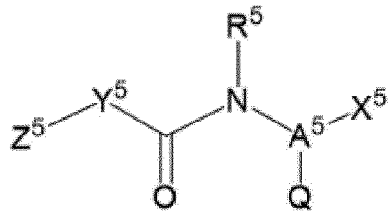

(5)

In the formula,

$A^5$ represents a trivalent hydrocarbon group having 1 or 2 carbon atoms, and
$R^5$ represents a hydrogen atom, an alkyl group having 1 to 10 carbon atoms, or a cycloalkyl group having 3 to 10 carbon atoms, each of which may contain -O-, -C(O)-, -OC(O)-, $-NJ^{51}-CO-$, $-CO-NJ^{51}-$, or -NH-CO-NH- in a molecular chain, $J^{51}$ represents a hydrogen atom or an alkyl group having 1 to 3 carbon atoms, and the alkyl group or the cycloalkyl group may have a substituent selected from a cycloalkyl group having 3 to 6 carbon atoms, an amino group, a cyano group, a mercapto group, a hydroxyl group, a carboxyl group, a phenyl group, a hydroxyphenyl group, a pyridyl group, a naphthyl group, a thienyl group, or a furyl group.
$X^5$ represents an alkyl group having one or more mercapto groups and having 1 to 10 carbon atoms, a cycloalkyl group having one or more mercapto groups and having 3 to 10 carbon atoms, an arylalkyl group having one or more mercapto groups and having 7 to 12 carbon atoms, a heteroalkylalkyl group having one or more mercapto groups and having 3 to 10 carbon atoms, or a mercapto group, each of which may contain -O-, -C(O)-, -OC(O)-, $-NJ^{52}-CO-$, $-CO-NJ^{52}-$, or -NH-CO-NH- in a molecular chain, $J^{52}$ represents a hydrogen atom or an alkyl group having 1 to 3 carbon atoms, and the alkyl group or the cycloalkyl group may have a substituent selected from a cycloalkyl group having 3 to 6 carbon atoms, an amino group, a cyano group, a hydroxyl group, a carboxyl group, a phenyl group, a hydroxyphenyl group, a pyridyl group, a naphthyl group, a thienyl group, or a furyl group.
$Y^5$ represents a group having 6 to 20 carbon atoms and containing a structure having an absorption spectrum in an ultraviolet, visible, or near-infrared region.
$Z^5$ represents $-CO-NR^{51}NR^{52}R^{53}$, where $R^{51}$, $R^{52}$, and $R^{53}$ each independently represent a hydrogen atom or an alkyl group having 1 to 10 carbon atoms.
Q represents a hydrogen atom, a carboxyl group, a hydroxyl group, or a primary amide structure, or represents $-CO-NR^{5a}NR^{5b}R^{5c}$, where $R^{5a}$, $R^{5b}$, and $R^{5c}$ each independently represent a hydrogen atom or an alkyl group having 1 to 10 carbon atoms.

<9> A compound represented by Formula (6).

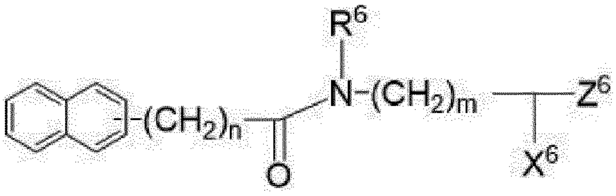

(6)

In the formula,

$R^6$ represents a hydrogen atom, an alkyl group having 1 to 10 carbon atoms, or a cycloalkyl group having 3 to 10 carbon atoms, each of which may contain -O-, -C(O)-, -OC(O)-, $-NJ^{61}-CO-$, $-CO-NJ^{61}-$, or -NH-CO-NH- in a molecular chain, $J^{61}$ represents a hydrogen atom or an alkyl group having 1 to 3 carbon atoms, and the alkyl group or the cycloalkyl group may have a substituent selected from a cycloalkyl group having 3 to 6 carbon atoms, an amino group, a cyano group, a mercapto group, a hydroxyl group, a carboxyl group, a phenyl group, a hydroxyphenyl group, a pyridyl group, a naphthyl group, a thienyl group, or a furyl group.
$X^6$ represents an alkyl group having one or more mercapto groups and having 1 to 10 carbon atoms, a cycloalkyl group having one or more mercapto groups and having 3 to 10 carbon atoms, an arylalkyl group having one or more mercapto groups and having 7 to 12 carbon atoms, a heteroalkylalkyl group having one or more mercapto groups and having 3 to 10 carbon atoms, or a mercapto group, each of which may contain -O-, -C(O)-, -OC(O)-, $-NJ^{62}-CO-$, $-CO-NJ^{62}-$, or -NH-CO-NH- in a molecular chain, $J^{62}$ represents a hydrogen atom or an alkyl group having 1 to 3 carbon atoms, and the alkyl group or the cycloalkyl group may have a substituent selected from a cycloalkyl group having 3 to 6 carbon atoms, an amino group, a cyano group, a hydroxyl group, a carboxyl group, a phenyl group, a hydroxyphenyl group, a pyridyl group, a naphthyl group, a thienyl group, or a furyl group.
$Z^6$ represents $-CO-NR^{61}NR^{62}R^{63}$, where $R^{61}$, $R^{62}$, and $R^{63}$ each independently represent a hydrogen atom or an alkyl group having 1 to 10 carbon atoms.
n represents 0 or 1, and
m represents 0 or 1.

<10> A compound represented by Formula (7).

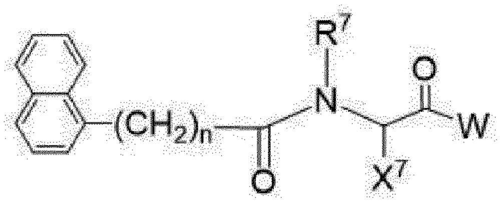

(7)

In the formula,

$R^7$ represents a hydrogen atom, an alkyl group having 1 to 10 carbon atoms, or a cycloalkyl group having 3 to 10 carbon atoms, each of which may contain -O-, -C(O)-, -OC(O)-, $-NJ^{71}-CO-$, $-CO-NJ^{71}-$, or -NH-CO-NH- in a molecular chain, $J^{71}$ represents a hydrogen atom or an alkyl group having 1 to 3 carbon atoms, and the alkyl group or the cycloalkyl group may have a substituent selected from a cycloalkyl group having 3 to 6 carbon atoms, an amino group, a cyano group, a mercapto group, a hydroxyl group, a carboxyl group, a phenyl group, a hydroxyphenyl group, a pyridyl group, a naphthyl group, a thienyl group, or a furyl group.
$X^7$ represents an alkyl group having one or more mercapto groups and having 1 to 10 carbon atoms, a cycloalkyl group having one or more mercapto groups and having 3 to 10 carbon atoms, an arylalkyl group having one or more mercapto groups and having 7 to 12 carbon atoms, a heteroalkylalkyl group having one or more mercapto groups and having 3 to 10 carbon atoms, or a mercapto group, each of which may contain -O-, -C(O)-, -OC(O)-, $-NJ^{72}-CO-$, $-CO-NJ^{72}-$, or -NH-CO-NH- in a molecular chain, $J^{72}$ represents a hydrogen atom or an alkyl group having 1 to 3 carbon atoms, and the alkyl group or the cycloalkyl group may have a substituent selected from a cycloalkyl group having 3 to 6 carbon atoms, an amino group, a cyano group, a hydroxyl group, a carboxyl group, a phenyl group, a hydroxyphenyl group, a pyridyl group, a naphthyl group, a thienyl group, or a furyl group.
W represents $NR^{71}-NR^{72}R^{73}$, where $R^{71}$, $R^{72}$, and $R^{73}$ represent a hydrogen atom or an alkyl group having 1 to 3 carbon atoms, and
n represents 0 or 1.

<11> A method for measuring skin sensitization comprising (1) reacting the reagent for measuring skin sensitization according to any one of <1> to <7> with a test substance, and (2) detecting an amount of the reagent for measuring skin sensitization after the reaction or an amount of a product of the reaction by optical measurement.

< 12> The method for measuring skin sensitization according to <11>, in which the test substance is at least one of a fragrance, an essential oil, a polymer compound, a pharmaceutical, an agricultural chemical, a food, a chemical product, or a plant extract consisting of a natural product-derived component.

<13> The method for measuring skin sensitization according to < 11 > or < 12>, further comprising subjecting a reaction product obtained in the step of reacting the reagent for measuring skin sensitization with the test substance to chromatography.

<14> The method for measuring skin sensitization according to any one of <11> to <13>, in which the optical measurement is a measurement using a fluorescence detector, an excitation wavelength is 200 to 600 nm, and a fluorescence wavelength is 200 to 800 nm.

According to an aspect of the present invention, it is possible to measure sensitization to a test substance with high sensitivity using a single type of reagent.

## BRIEF DESCRIPTION OF THE DRAWINGS

Fig. 1 shows the results of calculating a residual ratio of Compound 1 immediately after solution preparation (0 hours) and after 24 hours.

Fig. 2 shows the results of measuring a fluorescence intensity (peak area in HPLC) of Compound 1 immediately after solution preparation (0 hours).

Fig. 3 shows the results of comparing the depletions of nucleophilic reagents in No. 1 to No. 8 substances listed in Table 2.

Fig. 4 shows the results of comparing the depletions of nucleophilic reagents in No. 9 to No. 15 substances listed in Table 2.

## DESCRIPTION OF THE PREFERRED EMBODIMENTS

In the present specification, the expression "to" is used to include the numerical values before and after "to" as a lower limit value and an upper limit value, respectively.

In the present specification, the measurement of skin sensitization is meant to include the test of skin sensitization, and is also meant to include the determination of the presence or absence of skin sensitization based on a certain standard and the quantitative measurement of skin sensitization.

It is important that chemical substances contained in products such as pharmaceuticals, agricultural chemicals, and cosmetics do not have skin sensitization, and it is necessary to establish a method for predicting skin sensitization of chemical substances. Skin sensitization develops through a complex process consisting of many stages. The first event is the penetration of a test substance through the skin, followed by covalent bonding with a protein within the skin. Therefore, it is considered that evaluating this covalent bonding property makes it possible to predict whether or not the target test substance is skin sensitizing. It is known that the reaction between a protein in the skin and a test substance is caused by approximately five organic chemical reactions. It is known that the amino acids involved in these five reactions are the SH group of cysteine and the $NH_2$ group of lysine. Therefore, in the measurement of skin sensitization described in JP2011-59102A and JP2014-37995A, skin sensitization is predicted in such a manner that two types of nucleophilic reagents are chemically synthesized in which a naphthalene ring having a high molar absorption coefficient in an UV region is introduced at the N-terminals of cysteine and lysine, these two types of nucleophilic reagents are reacted with a test substance, and unreacted nucleophilic reagents are quantified to calculate the reactivity with the test substance.

In the present invention, the use of an organic compound having an absorption spectrum in an ultraviolet, visible, or near-infrared region makes it possible to quantify with dilute evaluation reagent and test substance concentrations, so precipitation due to poor dissolution does not occur and quantitativeness can be improved.

In the present invention, the use of an organic compound having a mercapto group and a hydrazide structure in the same molecule has made it possible to evaluate skin sensitization in a single operation.

The hydrazide group has a high reactivity with an aldehyde-based test substance having low sensitization, or exhibits high stability of a reaction product, so a false negative rate can be reduced in the present invention as compared with the conventional evaluation method which may result in false negative.

The reagent for measuring skin sensitization according to the embodiment of the present invention contains

an organic compound having a mercapto group and a hydrazide structure and having an absorption spectrum in an ultraviolet, visible, or near-infrared region, as a main measuring agent.

The organic compound used in the present invention is a compound which has an absorption spectrum in an ultraviolet, visible, or near-infrared region and exhibits absorption in a state as it is or in a solution state, preferably in a wavelength range of 190 to 2,500 nm and more preferably in a wavelength range of 200 to 700 nm.

The organic compound used in the present invention is preferably a compound having light emission at 200 to 800 nm, more preferably a compound having light emission at 200 to 700 nm, and still more preferably a compound having light emission at 250 to 650 nm.

The organic compound used in the present invention is preferably a compound represented by Formula (1) or Formula (2), and more preferably a compound represented by Formula (1).

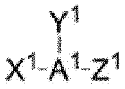 (1)

$X^2$-$Y^2$-$Z^2$ (2)

In the formulae,
$A^1$ represents a nitrogen atom or the following linking group.

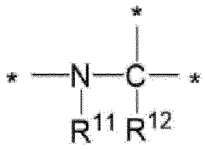
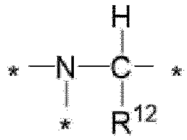
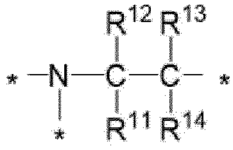
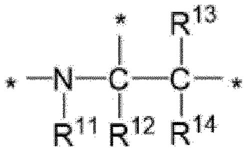
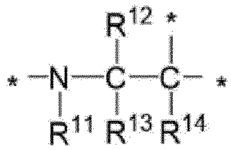

$R^{11}$, $R^{12}$, $R^{13}$, and $R^{14}$ each independently represent a hydrogen atom, an alkyl group having 1 to 10 carbon atoms, an alkenyl group having 2 to 10 carbon atoms, an alkynyl group having 2 to 10 carbon atoms, a cycloalkyl group having 3 to 10 carbon atoms, or a cycloalkenyl group having 3 to 10 carbon atoms, each of which may contain -O-, -C(O)-, -OC(O)-, -N$J^1$-CO-, -CO-N$J^1$-, or -NH-CO-NH- in a molecular chain, $J^1$ represents a hydrogen atom or an alkyl group having 1 to 3 carbon atoms, and the alkyl group, the alkenyl group, the alkynyl group, the cycloalkyl group, or the cycloalkenyl group may have a substituent selected from a cycloalkyl group having 3 to 6 carbon atoms, a cycloalkenyl group having 5 to 6 carbon atoms, an amino group, a cyano group, a mercapto group, a mercaptomethyl group, a hydroxyl group, a phenyl group, a hydroxyphenyl group, a pyridyl group, a naphthyl group, a thienyl group, or a furyl group.

**[0027]** * represents a connection position with $X^1$, $Y^1$, or $Z^1$.

$X^1$ and $X^2$ represent an alkyl group having one or more mercapto groups and having 1 to 10 carbon atoms, an alkenyl group having one or more mercapto groups and having 2 to 10 carbon atoms, an alkynyl group having one or more mercapto groups and having 2 to 10 carbon atoms, a cycloalkyl group having one or more mercapto groups and having 3 to 10 carbon atoms, a cycloalkenyl group having one or more mercapto groups and having 3 to 10 carbon atoms, an arylalkyl group having one or more mercapto groups and having 7 to 12 carbon atoms, a heteroalkylalkyl group having one or more mercapto groups and having 3 to 10 carbon atoms, or a mercapto group, each of which may contain -O-, -C(O)-, -OC(O)-, -N$J^2$-CO-, -CO-N$J^2$-, or -NH-CO-NH- in a molecular chain, $J^2$ represents an alkyl group having 1 to 3 carbon atoms, and the alkyl group, the alkenyl group, the alkynyl group, the cycloalkyl group, or the cycloalkenyl group may have a substituent selected from a cycloalkyl group having 3 to 6 carbon atoms, a cycloalkenyl group having 5 to 6 carbon atoms, an amino group, a cyano group, a mercaptomethyl group, a hydroxyl group, a phenyl group, a hydroxyphenyl group, a pyridyl group, a naphthyl group, a thienyl group, or a furyl group.

$Y^1$ and $Y^2$ represent a group having 6 to 20 carbon atoms and containing a structure having an absorption spectrum in an ultraviolet, visible, or near-infrared region.

$Z^1$ and $Z^2$ represent -CO-N$R^{21}$N$R^{22}R^{23}$, where $R^{21}$, $R^{22}$, and $R^{23}$ each independently represent a hydrogen atom or an alkyl group having 1 to 10 carbon atoms.

$R^{11}$, $R^{12}$, $R^{13}$, and $R^{14}$ are each independently preferably a hydrogen atom or an alkyl group having 1 to 10 carbon atoms.

$R^{11}$, $R^{12}$, $R^{13}$, and $R^{14}$ are each independently more preferably a hydrogen atom or a methyl group.
$R^{11}$, $R^{12}$, $R^{13}$, and $R^{14}$ are each independently particularly preferably a hydrogen atom.
$X^1$ and $X^2$ preferably represent an alkyl group having one or more mercapto groups and having 1 to 10 carbon atoms, an arylalkyl group having one or more mercapto groups and having 7 to 12 carbon atoms, a heteroalkylalkyl group having one or more mercapto groups and having 3 to 10 carbon atoms, or a mercapto group, each of which may contain -O-, -C(O)-, -OC(O)-, -$NJ^2$-CO-, -CO-$NJ^2$-, or -NH-CO-NH- in a molecular chain, and $J^2$ represents a hydrogen atom or an alkyl group having 1 to 3 carbon atoms.
$Y^1$ and $Y^2$ preferably represent a group having 10 to 20 carbon atoms and containing a structure having an absorption spectrum in an ultraviolet, visible, or near-infrared region.
$R^{21}$, $R^{22}$, and $R^{23}$ are each preferably a hydrogen atom.
The organic compound used in the present invention is still more preferably a compound represented by Formula (10).

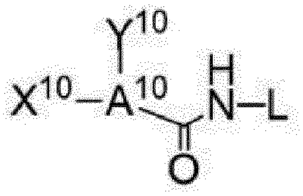 (10)

In the formula,

$A^{10}$ represents a nitrogen atom or a trivalent linking group, and
$X^{10}$ represents an alkyl group having one or more mercapto groups and having 1 to 10 carbon atoms, an alkenyl group having one or more mercapto groups and having 2 to 10 carbon atoms, an alkynyl group having one or more mercapto groups and having 2 to 10 carbon atoms, a cycloalkyl group having one or more mercapto groups and having 3 to 10 carbon atoms, a cycloalkenyl group having one or more mercapto groups and having 3 to 10 carbon atoms, an arylalkyl group having one or more mercapto groups and having 7 to 12 carbon atoms, a heteroalkylalkyl group having one or more mercapto groups and having 3 to 10 carbon atoms, or a mercapto group, each of which may contain -O-, -C(O)-, -OC(O)-, -$NJ^{101}$-CO-, -CO-$NJ^{101}$–, or -NH-CO-NH- in a molecular chain, $J^{101}$ represents a hydrogen atom or an alkyl group having 1 to 3 carbon atoms, and the alkyl group, the alkenyl group, the alkynyl group, the cycloalkyl group, or the cycloalkenyl group may have a substituent selected from a cycloalkyl group having 3 to 6 carbon atoms, a cycloalkenyl group having 5 to 6 carbon atoms, an amino group, a cyano group, a mercaptomethyl group, a hydroxyl group, a phenyl group, a hydroxyphenyl group, a pyridyl group, a naphthyl group, a thienyl group, or a furyl group.

$Y^{10}$ represents a group having 6 to 20 carbon atoms and containing a structure having an absorption spectrum in an ultraviolet, visible, or near-infrared region, and
L represents an amino group.
$A^{10}$ preferably represents a trivalent linking group, and more preferably

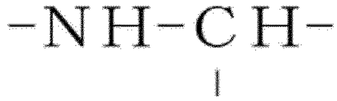 .

$X^{10}$ preferably represents an alkyl group having one or more mercapto groups and having 1 to 10 carbon atoms, an arylalkyl group having one or more mercapto groups and having 7 to 12 carbon atoms, a heteroalkylalkyl group having one or more mercapto groups and having 3 to 10 carbon atoms, or a mercapto group, each of which may contain -O-, -C(O)-, -OC(O)-, -$NJ^{101}$-CO-, -CO-$NJ^{101}$-, or -NH-CO-NH- in a molecular chain, and $J^{101}$ represents a hydrogen atom or an alkyl group having 1 to 3 carbon atoms.
$Y^{10}$ preferably represents a group having 10 to 20 carbon atoms and containing a structure having an absorption spectrum in an ultraviolet, visible, or near-infrared region.
The structure having an absorption spectrum in an ultraviolet, visible, or near-infrared region refers to a structure of a compound having absorption in a region from 200 nm to 2,500 nm. Examples of the compound having absorption in a region from 200 nm to 2,500 nm include a naphthalene derivative, an anthracene derivative, a phenanthrene derivative, a tetracene derivative, a pentacene derivative, a benzopyrene derivative, a chrysene derivative, a pyrene

derivative, a triphenylene derivative, a corannulene derivative, a coronene derivative, an ovalene derivative, an acridine derivative, a luciferin derivative, a pyranine derivative, a stilbene derivative, a benzofuran derivative, a dihydroquinoxalinone derivative, a phthalimidinyl derivative, a dansyl derivative, a merocyanine derivative, a perylene derivative, a rhodamine derivative, a coumarin derivative, a 4-(dicyanomethylene)-2-methyl-6-(4-dimethylaminostyryl)-4H-pyran (DCM) derivative, a pyrromethene derivative, a fluorescein derivative, an umbelliferone derivative, a benzothiazole derivative, a benzoxadiazole derivative, a shikonin derivative, a fluoranthene derivative, a carbazole derivative, a tetraphene derivative, an acenaphthene derivative, and a fluorene derivative. Specific examples of the compound having absorption in a region from 200 nm to 2,500 nm include compounds derived from 2-naphthylacetyl chloride, 4-(5,6-dimethoxy-N-phthalimidinyl)benzenesulfonic acid chloride (DPS-CL), 4-chloro-7-nitro-2,1,3-benzoxadiazole (NBD-CL), fluorescein isothiocyanate (FITC), rhodamine B isothiocyanate (RBITC), 4-fluoro-7-nitro-2,1,3-benzoxadiazole (NDB-F), 4-(N,N-dimethylaminosulfonyl)-7-fluoro-2,1,3-benzoxadiazole (DBD-F), 4-(N-phthalimidinyl)benzenesulfonic acid chloride (PHISYL-CL), 4-aminosulfonyl-7-fluoro-2,1,3-benzoxadiazole (ABD-F), N-[4-(6-dimethylamino-2-benzofuranyl)phenyl]maleimide (DBPM), 2-(4-maleimidephenyl)-6-methylbenzothiazole (MBPM), N-(9-acridinyl)maleimide (NAM), 4-chloro-7-sulfobenzofurazan ammonium salt (SBD-CL), 7-fluorobenzofurazan-4-sulfonic acid ammonium salt (SBD-F), 1,2-diamino-4,5-dimethoxybenzene (DDB), 4-(N,N-dimethylaminosulfonyl)-7-hydrazino-2,1,3-benzoxadiazole (DBD-H), 4-hydrazino-7-nitro-2,1,3-benzoxadiazolehydrazine (DBD-H), 2,2'-dithiodi(l-naphthylamine) (DTAN), 4-amino-3-penten-2-one (FLUORAL-P), 1,2-amino-4,5-methylenedioxybenzene (MDB), 4-(5,6-dimethoxybenzothiazol-2-yl)benzoic acid hydrazide (BHBT), 4-(N,N-dimethylaminosulfonyl)-7-(N-hydrazinocarbonylmethyl-N-methyl)amino-2,1,3-benzo xadiazole (DBD-CO-HZ), 4-(N-hydrazinocarbonylmethyl-N-methylamino)-7-nitro-2,1,3-benzoxadiazole (NBD-CO-HZ), 3-bromomethyl-6,7-dimethoxy-1-methyl-1,2-dihydroquinoxalin-2-one (BR-DMEQ), 4-bromomethyl-7-methoxycoumarin (BR-MMC), 4-(N,N-dimethylaminosulfonyl)-7-piperazino-2,1,3-benzoxadiazole (DBD-PZ), 4-nitro-7-piperazino-2,1,3-benzoxadiazole (NBD-PZ), 4-(N,N-dimethylaminosulfonyl)-7-(2-aminoethylamino)-2,1,3-benzoxadiazole (DBD-ED), 3-chlorocarbonyl-6,7-dimethoxy-1-methyl-2(1H)-quinoxalinone (DMEQ-COCL), 2-(5-chlorocarbonyl-2-oxazolyl)-5,6-methylenedioxybenzofuran (OMB-COCL), and the like.

The organic compound used in the present invention is even still more preferably a compound represented by Formula (3), Formula (4), or Formula (5). According to the present invention, the compound represented by Formula (3), Formula (4), or Formula (5) is provided.

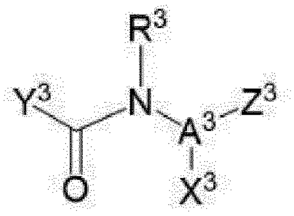
(3)

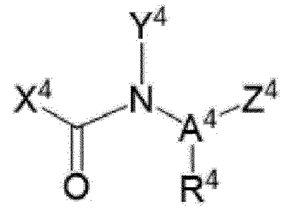
(4)

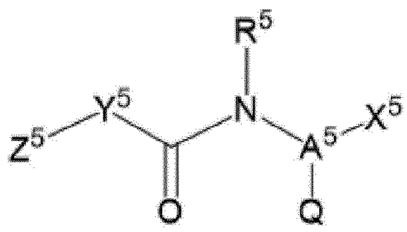
(5)

$A^3$, $A^4$, and $A^5$ represent a trivalent hydrocarbon group having 1 or 2 carbon atoms.

$R^3$, $R^4$, and $R^5$ represent a hydrogen atom, an alkyl group having 1 to 10 carbon atoms, or a cycloalkyl group having 3 to 10 carbon atoms, each of which may contain -O-, -C(O)-, -OC(O)-, $-NJ^{31}-CO-$, $-CO-NJ^{31}-$, or -NH-CO-NH- in a molecular chain, $J^{31}$ represents a hydrogen atom or an alkyl group having 1 to 3 carbon atoms, and the alkyl group or the cycloalkyl group may have a substituent selected from a cycloalkyl group having 3 to 6 carbon atoms, an amino

group, a cyano group, a mercapto group, a hydroxyl group, a carboxyl group, a phenyl group, a hydroxyphenyl group, a pyridyl group, a naphthyl group, a thienyl group, or a furyl group.

$X^3$, $X^4$, and $X^5$ represent an alkyl group having one or more mercapto groups and having 1 to 10 carbon atoms, a cycloalkyl group having one or more mercapto groups and having 3 to 10 carbon atoms, an arylalkyl group having one or more mercapto groups and having 7 to 12 carbon atoms, a heteroalkylalkyl group having one or more mercapto groups and having 3 to 10 carbon atoms, or a mercapto group, each of which may contain -O-, -C(O)-, -OC(O)-, -$NJ^{32}$-CO-, -CO-$NJ^{32}$-, or -NH-CO-NH- in a molecular chain, $J^{32}$ represents a hydrogen atom or an alkyl group having 1 to 3 carbon atoms, and the alkyl group or the cycloalkyl group may have a substituent selected from a cycloalkyl group having 3 to 6 carbon atoms, an amino group, a cyano group, a hydroxyl group, a carboxyl group, a phenyl group, a hydroxyphenyl group, a pyridyl group, a naphthyl group, a thienyl group, or a furyl group.

$Y^3$, $Y^4$, and $Y^5$ represent a group having 6 to 20 carbon atoms and containing a structure having an absorption spectrum in an ultraviolet, visible, or near-infrared region.

$Z^3$ represents -CO-$NR^{31}NR^{32}R^{33}$, where $R^{31}$, $R^{32}$, and $R^{33}$ each independently represent a hydrogen atom or an alkyl group having 1 to 10 carbon atoms.

$Z^4$ represents -CO-$NR^{41}NR^{42}R^{43}$, where $R^{41}$, $R^{42}$, and $R^{43}$ each independently represent a hydrogen atom or an alkyl group having 1 to 10 carbon atoms.

$Z^5$ represents -CO-$NR^{51}NR^{52}R^{53}$, where $R^{51}$, $R^{52}$, and $R^{53}$ each independently represent a hydrogen atom or an alkyl group having 1 to 10 carbon atoms.

Q represents a hydrogen atom, a carboxyl group, a hydroxyl group, or a primary amide structure, or represents -CO-$NR^{5a}NR^{5b}R^{5c}$, where $R^{5a}$, $R^{5b}$, and $R^{5c}$ each independently represent a hydrogen atom or an alkyl group having 1 to 10 carbon atoms.

$A^3$, $A^4$, and $A^5$ preferably represent

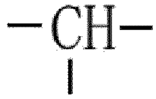

$R^3$, $R^4$, and $R^5$ preferably represent a hydrogen atom or an alkyl group having 1 to 10 carbon atoms, and more preferably a hydrogen atom or a methyl group.

$X^3$, $X^4$, and $X^5$ preferably represent an alkyl group having one or more mercapto groups and having 1 to 10 carbon atoms, an arylalkyl group having one or more mercapto groups and having 7 to 12 carbon atoms, a heteroalkylalkyl group having one or more mercapto groups and having 3 to 10 carbon atoms, or a mercapto group, each of which may contain -O-, -C(O)-, -OC(O)-, -$NJ^{32}$-CO-, -CO-$NJ^{32}$-, or -NH-CO-NH- in a molecular chain, and $J^{32}$ represents a hydrogen atom or an alkyl group having 1 to 3 carbon atoms.

$Y^3$, $Y^4$, and $Y^5$ preferably represent a group having 10 to 20 carbon atoms and containing a structure having an absorption spectrum in an ultraviolet, visible, or near-infrared region.

$R^{21}$, $R^{22}$, and $R^{23}$ preferably represent a hydrogen atom.

Q preferably represents a primary amide structure.

In the present invention, $Y^1$, $Y^2$, $Y^3$, $Y^4$, $Y^5$, and $Y^{10}$ are preferably groups that emit fluorescence.

The organic compound used in the present invention is even further still more preferably a compound represented by Formula (6). According to the present invention, the compound represented by Formula (6) is provided.

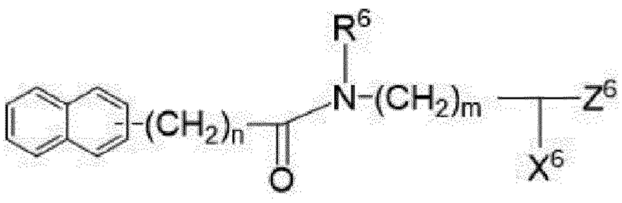
(6)

$R^6$ represents a hydrogen atom, an alkyl group having 1 to 10 carbon atoms, or a cycloalkyl group having 3 to 10 carbon atoms, each of which may contain -O-, -C(O)-, -OC(O)-, -$NJ^{61}$-CO-, -CO-$NJ^{61}$-, or -NH-CO-NH- in a molecular chain, $J^{61}$ represents a hydrogen atom or an alkyl group having 1 to 3 carbon atoms, and the alkyl group or the cycloalkyl group may have a substituent selected from a cycloalkyl group having 3 to 6 carbon atoms, an amino group, a cyano group, a mercapto group, a hydroxyl group, a carboxyl group, a phenyl group, a hydroxyphenyl group, a pyridyl group, a naphthyl group, a thienyl group, or a furyl group.

$X^6$ represents an alkyl group having one or more mercapto groups and having 1 to 10 carbon atoms, a cycloalkyl

group having one or more mercapto groups and having 3 to 10 carbon atoms, an arylalkyl group having one or more mercapto groups and having 7 to 12 carbon atoms, a heteroalkylalkyl group having one or more mercapto groups and having 3 to 10 carbon atoms, or a mercapto group, each of which may contain -O-, -C(O)-, -OC(O)-, $-NJ^{62}-CO-$, $-CO-NJ^{62}-$, or -NH-CO-NH- in a molecular chain, $J^{62}$ represents a hydrogen atom or an alkyl group having 1 to 3 carbon atoms, and the alkyl group or the cycloalkyl group may have a substituent selected from a cycloalkyl group having 3 to 6 carbon atoms, an amino group, a cyano group, a hydroxyl group, a carboxyl group, a phenyl group, a hydroxyphenyl group, a pyridyl group, a naphthyl group, a thienyl group, or a furyl group.

$Z^6$ represents $-CO-NR^{61}NR^{62}R^{63}$, where $R^{61}$, $R^{62}$, and $R^{63}$ each independently represent a hydrogen atom or an alkyl group having 1 to 10 carbon atoms.

n represents 0 or 1, and
m represents 0 or 1.

$R^6$ preferably represents a hydrogen atom or a methyl group.

$X^6$ preferably represents an alkyl group having one or more mercapto groups and having 1 to 10 carbon atoms, an arylalkyl group having one or more mercapto groups and having 7 to 12 carbon atoms, a heteroalkylalkyl group having one or more mercapto groups and having 3 to 10 carbon atoms, or a mercapto group, each of which may contain -O-, -C(O)-, -OC(O)-, $-NJ^{62}-CO-$, $-CO-NJ^{62}-$, or -NH-CO-NH- in a molecular chain, and $J^{62}$ represents a hydrogen atom or an alkyl group having 1 to 3 carbon atoms.

$R^{61}$, $R^{62}$, and $R^{63}$ preferably represent a hydrogen atom.

n preferably represents 1.
m preferably represents 0.

The organic compound used in the present invention is particularly preferably a compound represented by Formula (7). According to the present invention, the compound represented by Formula (7) is provided.

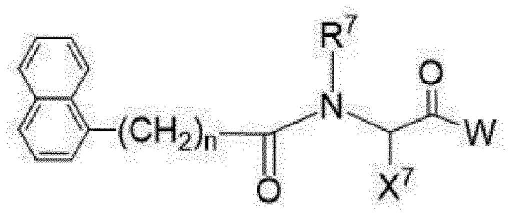

(7)

$R^7$ represents a hydrogen atom, an alkyl group having 1 to 10 carbon atoms, or a cycloalkyl group having 3 to 10 carbon atoms, each of which may contain -O-, -C(O)-, -OC(O)-, $-NJ^{71}-CO-$, $-CO-NJ^{71}-$, or -NH-CO-NH- in a molecular chain, $J^{71}$ represents a hydrogen atom or an alkyl group having 1 to 3 carbon atoms, and the alkyl group or the cycloalkyl group may have a substituent selected from a cycloalkyl group having 3 to 6 carbon atoms, an amino group, a cyano group, a mercapto group, a hydroxyl group, a carboxyl group, a phenyl group, a hydroxyphenyl group, a pyridyl group, a naphthyl group, a thienyl group, or a furyl group.

$X^7$ represents an alkyl group having one or more mercapto groups and having 1 to 10 carbon atoms, a cycloalkyl group having one or more mercapto groups and having 3 to 10 carbon atoms, an arylalkyl group having one or more mercapto groups and having 7 to 12 carbon atoms, a heteroalkylalkyl group having one or more mercapto groups and having 3 to 10 carbon atoms, or a mercapto group, each of which may contain -O-, -C(O)-, -OC(O)-, $-NJ^{72}-CO-$, $-CO-NJ^{72}-$, or -NH-CO-NH- in a molecular chain, $J^{72}$ represents a hydrogen atom or an alkyl group having 1 to 3 carbon atoms, and the alkyl group or the cycloalkyl group may have a substituent selected from a cycloalkyl group having 3 to 6 carbon atoms, an amino group, a cyano group, a hydroxyl group, a carboxyl group, a phenyl group, a hydroxyphenyl group, a pyridyl group, a naphthyl group, a thienyl group, or a furyl group.

W represents $NR^{71}-NR^{72}R^{73}$, where $R^{71}$, $R^{72}$, and $R^{73}$ represent a hydrogen atom or an alkyl group having 1 to 3 carbon atoms, and

n represents 0 or 1.

$R^7$ preferably represents a hydrogen atom or a methyl group.

$X^7$ preferably represents an alkyl group having one or more mercapto groups and having 1 to 10 carbon atoms, an arylalkyl group having one or more mercapto groups and having 7 to 12 carbon atoms, a heteroalkylalkyl group having one or more mercapto groups and having 3 to 10 carbon atoms, or a mercapto group, each of which may contain -O-, -C(O)-, -OC(O)-, $-NJ^{72}-CO-$, $-CO-NJ^{72}-$, or -NH-CO-NH- in a molecular chain, and $J^{72}$ represents a hydrogen atom or an alkyl group having 1 to 3 carbon atoms.

$R^{71}$, $R^{72}$, and $R^{73}$ preferably represent a hydrogen atom.
n preferably represents 1.

Examples of the alkyl group having 1 to 10 carbon atoms include a methyl group, an ethyl group, a propyl group, an isopropyl group, a butyl group, an isobutyl group, a sec-butyl group, a tert-butyl group, a pentyl group, an isopentyl group, a neopentyl group, a tert-pentyl group, a hexyl group, a heptyl group, an octyl group, a nonyl group, and a decyl group.

Examples of the alkenyl group having 2 to 10 carbon atoms include a vinyl group, a 1-propenyl group, a 2-propenyl group, a 1-butenyl group, a 2-butenyl group, a 3-butenyl group, a 1,3-butenyl group, a 1-pentenyl group, a 1-hexenyl group, a 1-heptenyl group, a 1-octenyl group, a 1-nonenyl group, and a 1-decenyl group.

Examples of the alkynyl group having 2 to 10 carbon atoms include an ethynyl group, a 1-propynyl group, a 1-butynyl group, a 1-pentynyl group, a 1-hexynyl group, a 1-heptynyl group, a 1-octynyl group, a 1-noninyl group, and a 1-decynyl group.

Examples of the cycloalkyl group having 3 to 10 carbon atoms include a cyclopropyl group, a cyclobutyl group, a cyclopentyl group, a cyclohexyl group, a cycloheptyl group, a cyclooctyl group, a cyclononyl group, and a cyclodecyl group.

Examples of the cycloalkenyl group having 3 to 10 carbon atoms include a cyclopropenyl group, a cyclobutenyl group, a cyclopentenyl group, a cyclohexenyl group, a cycloheptenyl group, a cyclooctenyl group, a cyclononenyl group, and a cyclodecenyl group.

Examples of the arylalkyl group having 7 to 12 carbon atoms include a phenylmethyl group and a phenylethyl group.

Examples of the heteroalkylalkyl group having 3 to 10 carbon atoms include the following structures. * represents a bonding point. In a case of having an asymmetric carbon in the structure, the heteroalkylalkyl group having 3 to 10 carbon atoms includes all possible stereoisomers.

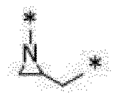
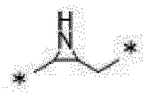
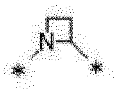
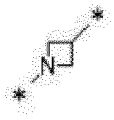
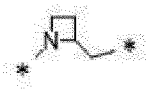
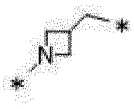

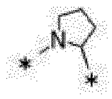
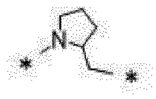
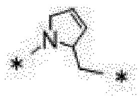
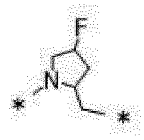
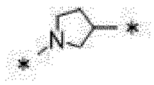
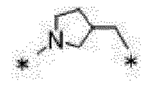

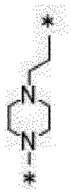
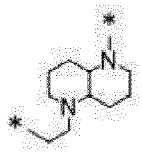
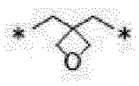
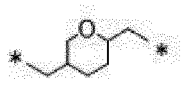
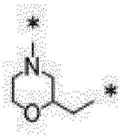

<Synthesis method of organic compound>

The organic compound used in the present invention can be produced by a chemical synthesis method. As an example, Compound 1 described in Examples can be synthesized by reacting 1-naphthylacetic acid with S-trityl-L-cysteine in the presence of 1,1'-carbonyldiimidazole to produce an intermediate N-(2-(naphthalen-1-yl)acetyl)-S-trityl-L-cysteine and then reacting this intermediate with hydrazine monohydrate in the presence of 1,1'-carbonyldiimidazole.

Alternatively, the organic compound used in the present invention can be produced by using a known peptide synthesis method. Specifically, the organic compound used in the present invention can be produced according to the method described in the synthesis of Compounds 6 to 12 and 14 in Examples which will be described later. That is, the organic compound used in the present invention can be synthesized by carrying out solid phase peptide synthesis using a commercially available automatic peptide synthesizer.

A resin for solid phase synthesis, N-methyl-2-pyrrolidone (NMP) solutions of Fmoc amino acids, an NMP solution of ethyl cyanohydroxyiminoacetate, an NMP solution of diisopropylethylamine, an NMP solution of diisopropylcarbodiimide, an NMP solution of piperidine, and an NMP solution of anhydrous acetic acid can be set in a synthesizer for synthesis. A cycle of Fmoc deprotection, washing with NMP, condensation of Fmoc amino acids, and washing with NMP

is repeated, whereby the peptide chain can be elongated.

<Reagent for measuring skin sensitization>

The reagent for measuring skin sensitization according to the embodiment of the present invention may consist only of the above-mentioned organic compound, or may contain one or two or more additives in addition to the above-mentioned organic compound which is a main measuring agent. Examples of the additive include a pH adjuster and a stabilizer. In addition, the reagent for measuring skin sensitization according to the embodiment of the present invention may be obtained by dissolving the above-mentioned main measuring agent and, if necessary, the above-mentioned additives in water, an aqueous buffer solution, an organic solvent, a mixed solvent of any of these, or the like.

The reagent for measuring skin sensitization according to the embodiment of the present invention may be provided in any form of a solution, a liquid, or a solid (a powder, a granule, a freeze-dried product, a tablet, or the like.).

<Method for measuring skin sensitization>

The method for measuring skin sensitization according to the embodiment of the present invention includes

(1) reacting the reagent for measuring skin sensitization according to the embodiment of the present invention with a test substance, and
(2) detecting an amount of the reagent for measuring skin sensitization after the reaction or an amount of a product of the reaction by optical measurement.

The reagent for measuring skin sensitization according to the embodiment of the present invention may be used at a concentration of the organic compound of, for example, about 0.01 μmol/L to about 1 mol/L and usually about 1 μmol/L to about 100 μmol/L, for example, in the form of being dissolved in an aqueous buffer solution such as a phosphate buffer solution or an organic solvent such as dimethyl sulfoxide (DMSO) and further diluted with an aqueous buffer solution such as a phosphate buffer solution or another organic solvent as necessary.

The type of the test substance is not particularly limited, and is, for example, at least one of a fragrance, an essential oil, a polymer compound, a pharmaceutical, an agricultural chemical, a food, a chemical product, or a plant extract consisting of a natural product-derived component. The test substance may be dissolved in, for example, water, an organic solvent such as methanol, ethanol, acetonitrile, acetone, or dimethyl sulfoxide (DMSO), or a mixed solvent thereof to, for example, a concentration of about 0.01 μmol/L to about 1 mol/L and usually a concentration of about 0.1 mmol/L to about 500 mmol/L. For the purpose of preventing precipitation of the test substance, the test substance may be preferably dissolved at a concentration of 0.1 mmol/L to 100 mmol/L and more preferably 0.1 mmol/L to 10 mmol/L.

The above-mentioned organic compound which is the main measuring agent of the reagent for measuring skin sensitization according to the embodiment of the present invention and the test substance solution may be mixed and reacted so that the molar concentration ratio of the organic compound and the test substance is, for example, 1:200 to 10:1. The reaction can carried out in such a manner that a solution containing the above-mentioned organic compound and the test substance is stirred or allowed to stand usually for about 1 minute to about 2 days while keeping the temperature in a temperature range of, for example, about 4°C to about 60°C.

The skin sensitization of the test substance can be measured by examining the reactivity between the organic compound and the test substance by the above reaction. In order to examine the above-mentioned reactivity, the residual amount of the above-mentioned organic compound and/or the produced amount of a reaction product between the above-mentioned organic compound and the test substance in a mixed solution of the reagent for measuring skin sensitization solution and the test substance solution may be analyzed. By carrying out this analysis over time, the skin sensitization of the test substance can be evaluated by obtaining the reaction rate constants of the above-mentioned organic compound and the test substance and comparing the reaction rate constants of different test substances or by comparing the reaction rate constant of the test substance with the reaction rate constant obtained for a compound whose presence or absence and strength of skin sensitization have been confirmed in animal experiments.

In a case of analyzing the residual amount, and then in a case where there is a possibility that the reagent for measuring skin sensitization may cause some change in the reaction solution, if necessary, a reaction solution (control group) that does not contain only the test substance may be separately prepared and analyzed, and then the correction may be made based on the value of the residual amount in this reaction solution.

The method according to the embodiment of the present invention may include subjecting a reaction product obtained in the step of reacting the reagent for measuring skin sensitization with the test substance to chromatography. That is, the method for analyzing a compound and the compound produced by the above reaction is not particularly limited. For example, the compound produced by the above reaction, the above-mentioned organic compound, and the test substance can be separated and analyzed by high performance liquid chromatography (HPLC), gas chromatography

(GC), thin layer chromatography (TLC), or the like.

Examples of chromatography modes that can be used for the HPLC, GC, or TLC include reverse phase, normal phase, and ion exchange. Examples of commercially available columns and TLCs that can be used for such chromatography modes include LC columns such as CAPCELL-PAK (manufactured by Osaka Soda Co., Ltd.), L-column ODS (manufactured by Chemicals Evaluation and Research Institute, Japan), and Shodex Asahipak (manufactured by Showa Denko K.K.), and TLC plates such as silica gel 60F254 (manufactured by Merck & Co., Inc.) and Silica Gel Plate (manufactured by Nacalai Tesque, Inc.).

The method for detecting the compound produced by the above reaction or the remaining organic compound is not particularly limited, and examples of the detector that can be used in the HPLC analysis include a UV-Vis detector, a near-infrared detector, a fluorescence detector, a differential refractive index detector, an electrical conductivity detector, and an evaporative light scattering detector. Examples of the UV-Vis detector include a single wavelength UV-Vis detector, a dual wavelength UV-Vis detector, and a photodiode array detector. In addition, examples of commercially available detectors that can be used for such a detection method include UV-Vis detectors, differential refractive index detectors, and electrical conductivity detectors manufactured by Shimadzu Corporation, Hitachi, Ltd., Waters Corporation, and Shiseido Co., Ltd., and evaporative light scattering detectors manufactured by Shimadzu Corporation.

In an example of the present invention, % depletion of the reagent for measuring skin sensitization (also referred to as a nucleophilic reagent) after the reaction between the test substance and the reagent for measuring skin sensitization may be detected by optical measurement using an ultraviolet detector. A commercially available detector can be used as the ultraviolet detector, and examples thereof include ultraviolet detectors manufactured by Shimadzu Corporation, Waters Corporation, Hitachi, Ltd., and Agilent Technologies, Inc.

In the optical measurement using an ultraviolet detector, the detection wavelength is preferably 200 to 700 nm, more preferably 200 to 600 nm, still more preferably 220 to 550 nm, and even still more preferably 280 to 480 nm.

In another example of the present invention, % depletion of the reagent for measuring skin sensitization (also referred to as a nucleophilic reagent) after the reaction between the test substance and the reagent for measuring skin sensitization may be detected by optical measurement using a fluorescence detector.

A molecule in a ground state absorbs excitation light and transitions to an excited state. Part of the absorbed excitation energy is deactivated by vibration energy or the like, and the light emitted in a case of returning to the ground state after a non-radiative transition to a position where a vibration level is low is fluorescence. The optical measurement using a fluorescence detector is generally said to be an analytical technique with a sensitivity that is $10^3$ times or more higher than that of absorptiometry. Further, since the optical measurement using a fluorescence detector is intended for measurement of a fluorescent substance, it is excellent in selectivity and is used as a technique for analysis of extremely small amounts. Since the fluorescence intensity is proportional to the concentration of the fluorescent substance, quantitative analysis can be carried out by creating a calibration curve. A commercially available detector can be used as the fluorescence detector, and examples thereof include fluorescence detectors manufactured by Shimadzu Corporation, Waters Corporation, Hitachi, Ltd., Agilent Technologies, Inc., and Osaka Soda Co., Ltd.

In the optical measurement using a fluorescence detector, the excitation wavelength is preferably 200 to 800 nm, more preferably 200 to 600 nm, still more preferably 200 to 550 nm, even still more preferably 200 to 500 nm, and particularly preferably 200 to 480 m. The fluorescence wavelength is preferably 200 to 1,000 nm, more preferably 200 to 800 nm, still more preferably 200 to 700 nm, and particularly preferably 200 to 650 nm.

% depletion of the reagent for measuring skin sensitization (also referred to as a nucleophilic reagent) can be calculated according to the following expression from an average value of peak areas of the reagent for measuring skin sensitization (also referred to as a nucleophilic reagent) in the optical measurement using an ultraviolet detector or a fluorescence detector.

% depletion of nucleophilic reagent = [1 - (average value of peak areas of unreacted nucleophilic reagent after reaction/average value of peak areas of standard nucleophilic reagent)] × 100

The detection in the measurement method using the reagent for measuring skin sensitization according to the embodiment of the present invention is not limited to the above. For example, the detection may be carried out by detecting an ion having a specific mass based on a molecular weight or the like with reference to the method described in JP2003-14761A or JP2008-139275A.

Hereinafter, the present invention will be described in detail with reference to Examples, but the present invention is not limited thereto.

Examples

<Explanation of terms>

EDTA: ethylenediamine tetraacetic acid
TFA: trifluoroacetic acid
DMSO: dimethyl sulfoxide
NMP: N-methyl-2-pyrrolidone

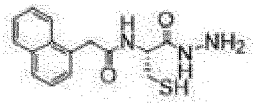

Compound 1

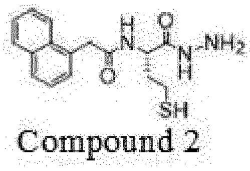

Compound 2

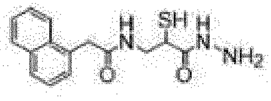

Compound 3

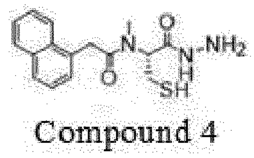

Compound 4

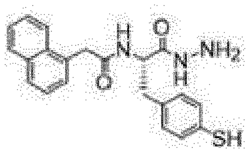

Compound 5

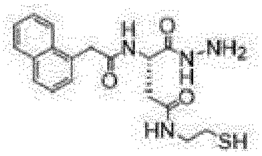

Compound 6

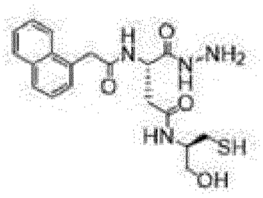

Compound 7

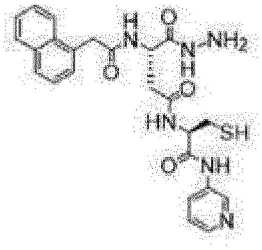

Compound 8

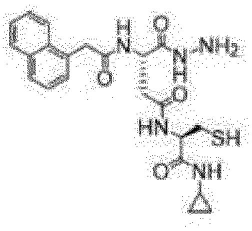

Compound 9

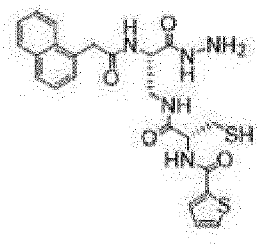

Compound 10

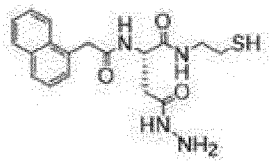

Compound 11

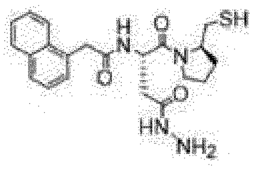

Compound 12

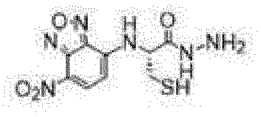

Compound 13

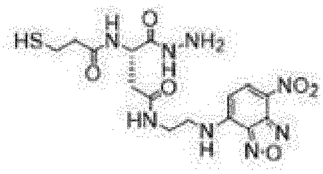

Compound 14

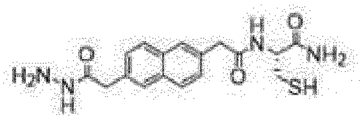

Compound 15

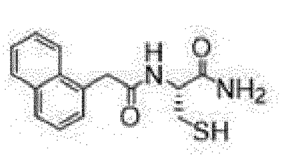

Comparative Example 1

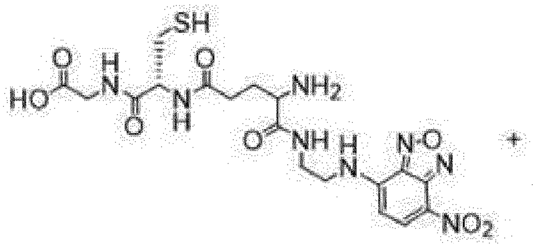

+

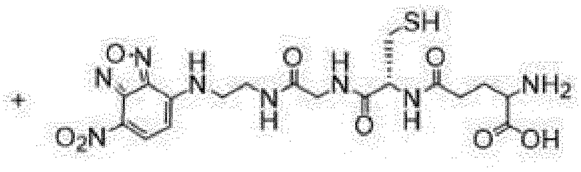

Comparative Example 2

(Synthesis of Compound 1)

269 mg of 1-naphthylacetic acid (manufactured by FUJIFILM Wako Pure Chemical Corporation) and 10 mL of dimethylformamide (manufactured by FUJIFILM Wako Pure Chemical Corporation) were placed and dissolved in an eggplant flask to which 234 mg of 1,1'-carbonyldiimidazole (manufactured by FUJIFILM Wako Pure Chemical Corporation) was then added, followed by stirring for 2 hours. Then, 500 mg of S-trityl-L-cysteine (Cys(Trt)-OH) (manufactured by Tokyo Chemical Industry Co., Ltd.) and 250 μL of N,N-diisopropylethylamine (manufactured by FUJIFILM Wako Pure Chemical Corporation) were added thereto, followed by stirring for 2 hours. After completion of the reaction, water was added to the reaction solution, followed by extraction with ethyl acetate (manufactured by FUJIFILM Wako Pure Chemical Corporation), and the organic layer was washed with water and saturated saline and then dried over anhydrous sodium sulfate (manufactured by FUJIFILM Wako Pure Chemical Corporation). After removing the anhydrous sodium sulfate by filtration, the filtrate was distilled off under vacuum to obtain 690 mg of an intermediate crude product N-(2-(naphthalen-1-yl)acetyl)-S-trityl-L-cysteine.

Next, 690 mg of the intermediate crude product and 10 mL of dimethylformamide (manufactured by FUJIFILM Wako Pure Chemical Corporation) were placed and dissolved in an eggplant flask to which 230 mg of 1,1'-carbonyldiimidazole (manufactured by FUJIFILM Wako Pure Chemical Corporation) was then added, followed by stirring for 2 hours. Next, 72 mg of hydrazine monohydrate (manufactured by FUJIFILM Wako Pure Chemical Corporation) was added thereto, followed by stirring for 2 hours. Then, water was added to the reaction solution, followed by extraction with ethyl acetate (manufactured by FUJIFILM Wako Pure Chemical Corporation), and the organic layer was washed with water and saturated saline and then dried over anhydrous sodium sulfate (manufactured by FUJIFILM Wako Pure Chemical Corporation). After removing the anhydrous sodium sulfate by filtration, the filtrate was distilled off under vacuum.

Next, 2 mL of trifluoroacetic acid (TFA) (manufactured by FUJIFILM Wako Pure Chemical Corporation):triisopropylsilane (manufactured by Tokyo Chemical Industry Co., Ltd.):water (= 95:2.5:2.5) was added to the distillate. After stirring for 2 hours, the solvent was distilled off under reduced pressure. The resulting residue was purified by liquid chromatography and then the solvent was distilled off under reduced pressure, followed by freeze-drying to obtain 95 mg of a white solid (Compound 1).
Observed MS (ESI m/z): 304.3 (M + H), RT (min): 1.03

(Synthesis of Compound 2 to Compound 5)

Compound 2 to Compound 5 were synthesized according to the synthesis method of Compound 1.
Compound 2 was synthesized according to the synthesis method of Compound 1, except that S-trityl-L-homocysteine (synthesized by the method described in the literature, Journal of Medicinal Chemistry, 1996, vol. 39, # 7, p. 136) was used instead of S-trityl-L-cysteine (Cys(Trt)-OH) used in the synthesis of Compound 1.
Compound 3 was synthesized according to the synthesis method of Compound 1, except that S-trityl-isocysteine (synthesized by the method described in the literature, Bioorganic and Medicinal Chemistry, 2008, vol. 16, # 1, p. 65) was used instead of S-trityl-L-cysteine (Cys(Trt)-OH) used in the synthesis of Compound 1.
Compound 4 was synthesized according to the synthesis method of Compound 1, except that (2R)-2-(methylamino)-3-[(triphenylmethyl)sulfanyl]propanoic acid (manufactured by ChemShuttle, Inc.) was used instead of S-trityl-L-cysteine (Cys(Trt)-OH) used in the synthesis of Compound 1.

Compound 5 was synthesized according to the synthesis method of Compound 1, except that 4-mercaptophenylalanine (manufactured by Chemspace Ltd.) was used instead of S-trityl-L-cysteine (Cys(Trt)-OH) used in the synthesis of Compound 1.

(Synthesis of Compound 6)

Solid phase peptide synthesis was carried out using 2-chlorotrityl chloride resin (manufactured by Watanabe Chemical Industries, Ltd.) as a resin for solid phase synthesis. The resin was used in an amount equivalent to 0.05 mmol. 0.075 mmol of N-α-(9-fluorenylmethoxycarbonyl)-L-aspartic acid β-allyl ester (manufactured by Watanabe Chemical Industries, Ltd.) adjusted with a 0.5 mol/L methylene chloride solution and 0.4 mL of diisopropylethylamine (manufactured by Tokyo Chemical Industry Co., Ltd.) were added to the resin swollen with methylene chloride (manufactured by FUJIFILM Wako Pure Chemical Corporation), followed by shaking for 2 hours. The reaction was followed by washing with methylene chloride and N-methyl-2-pyrrolidone (manufactured by FUJIFILM Wako Pure Chemical Corporation). Next, after condensation of 1-naphthylacetic acid (manufactured by FUJIFILM Wako Pure Chemical Corporation) and deprotection of the allyl group, 2-[(triphenylmethyl)sulfanyl]ethanamine (manufactured by Combi-Blocks Inc.) was condensed. After completion of the peptide synthesis, the resin was washed with dichloromethane (manufactured by FUJIFILM Wako Pure Chemical Corporation), and then the solvent was distilled off under reduced pressure. 2 mL of trifluoroacetic acid (TFA) (manufactured by FUJIFILM Wako Pure Chemical Corporation) :triisopropylsilane (manufactured by Tokyo Chemical Industry Co., Ltd.):water (= 95:2.5:2.5) was added to cleave the peptide from the resin while simultaneously carrying out deprotection. After 2 hours, the resin was filtered off, and 12 mL of n-hexane (manufactured by FUJIFILM Wako Pure Chemical Corporation):methyl-t-butyl ether (manufactured by FUJIFILM Wako Pure Chemical Corporation) (= 1:1) was added to the filtrate to generate a solid. The solid was precipitated by centrifugation and then the supernatant was removed. The solid was washed with methyl-t-butyl ether (manufactured by FUJIFILM Wako Pure Chemical Corporation), and then the solvent was distilled off under reduced pressure. Next, the intermediate crude product was dissolved in 2 mL of dimethylformamide (manufactured by FUJIFILM Wako Pure Chemical Corporation) to which 32 mg (0.2 mmol) of 1,1'-carbonyldiimidazole (manufactured by FUJIFILM Wako Pure Chemical Corporation) was then added, followed by stirring for 2 hours. Next, 25 mg (0.5 mmol) of hydrazine monohydrate (manufactured by FUJIFILM Wako Pure Chemical Corporation) was added thereto, followed by stirring for 2 hours. Then, water was added to the reaction solution, followed by extraction with ethyl acetate (manufactured by FUJIFILM Wako Pure Chemical Corporation), and the organic layer was washed with water and saturated saline and then dried over anhydrous sodium sulfate (manufactured by FUJIFILM Wako Pure Chemical Corporation). After removing the anhydrous sodium sulfate by filtration, the filtrate was distilled off under vacuum. The resulting residue was purified by liquid chromatography and then the solvent was distilled off under reduced pressure, followed by freeze-drying to obtain a white solid.

(Synthesis of Compound 7)

Compound 7 was synthesized according to the synthesis method of Compound 6, except that (R)-2-amino-3-(tritylthio)propan-1-ol (manufactured by AstaTech, Inc.) was used instead of 2-[(triphenylmethyl)sulfanyl]ethanamine used in the synthesis of Compound 6.

(Synthesis of Compound 8)

Solid phase peptide synthesis was carried out using 2-chlorotrityl chloride resin (manufactured by Watanabe Chemical Industries, Ltd.) as a resin for solid phase synthesis. The resin was used in an amount equivalent to 0.05 mmol. 0.075 mmol of N-α-(9-fluorenylmethoxycarbonyl)-L-aspartic acid β-allyl ester (synthesized by the method described in the literature, Organic Letters, 2013, vol. 15, # 19, p. 5076) adjusted with a 0.5 mol/L methylene chloride solution and 0.4 mL of diisopropylethylamine (manufactured by Tokyo Chemical Industry Co., Ltd.) were added to the resin swollen with methylene chloride (manufactured by FUJIFILM Wako Pure Chemical Corporation), followed by shaking for 2 hours. The reaction was followed by washing with methylene chloride and N-methyl-2-pyrrolidone (manufactured by FUJIFILM Wako Pure Chemical Corporation). Next, after condensation of 1-naphthylacetic acid (manufactured by FUJIFILM Wako Pure Chemical Corporation) and deprotection of the allyl group, condensation of S-trityl-L-cysteine-allyl ester (synthesized by the method described in the literature, Organic Letters, 2013, vol. 15, # 19, p. 5076) and deprotection of the allyl group were carried out, followed by further condensation of 3-aminopyridine (manufactured by FUJIFILM Wako Pure Chemical Corporation).

After completion of the peptide synthesis, the resin was washed with dichloromethane (manufactured by FUJIFILM Wako Pure Chemical Corporation), and then the solvent was distilled off under reduced pressure. 2 mL of trifluoroacetic acid (TFA) (manufactured by FUJIFILM Wako Pure Chemical Corporation):triisopropylsilane (manufactured by Tokyo Chemical Industry Co., Ltd.):water (= 95:2.5:2.5) was added to cleave the peptide from the resin while

simultaneously carrying out deprotection. After 2 hours, the resin was filtered off, and 12 mL of n-hexane (manufactured by FUJIFILM Wako Pure Chemical Corporation):methyl-t-butyl ether (manufactured by FUJIFILM Wako Pure Chemical Corporation) (= 1:1) was added to the filtrate to generate a solid. The solid was precipitated by centrifugation and then the supernatant was removed. The solid was washed with methyl-t-butyl ether (manufactured by FUJIFILM Wako Pure Chemical Corporation), and then the solvent was distilled off under reduced pressure. Next, the intermediate crude product was dissolved in 2 mL of dimethylformamide (manufactured by FUJIFILM Wako Pure Chemical Corporation) to which 32 mg (0.2 mmol) of 1,1'-carbonyldiimidazole (manufactured by FUJIFILM Wako Pure Chemical Corporation) was then added, followed by stirring for 2 hours. Next, 25 mg (0.5 mmol) of hydrazine monohydrate (manufactured by FUJIFILM Wako Pure Chemical Corporation) was added thereto, followed by stirring for 2 hours. Then, water was added to the reaction solution, followed by extraction with ethyl acetate (manufactured by FUJIFILM Wako Pure Chemical Corporation), and the organic layer was washed with water and saturated saline and then dried over anhydrous sodium sulfate (manufactured by FUJIFILM Wako Pure Chemical Corporation). After removing the anhydrous sodium sulfate by filtration, the filtrate was distilled off under vacuum. The resulting residue was purified by liquid chromatography and then the solvent was distilled off under reduced pressure, followed by freeze-drying to obtain a white solid.

(Synthesis of Compound 9)

Compound 9 was synthesized according to the synthesis method of Compound 8, except that cyclopropylamine (manufactured by Tokyo Chemical Industry Co., Ltd.) was used instead of 3-aminopyridine used in the synthesis of Compound 8.

(Synthesis of Compound 10)

Solid phase peptide synthesis was carried out using 2-chlorotrityl chloride resin (manufactured by Watanabe Chemical Industries, Ltd.) as a resin for solid phase synthesis. The resin was used in an amount equivalent to 0.05 mmol. 0.075 mmol of N-α-Fmoc-N-β-alloc-L-diaminopropionic acid (manufactured by Iris Biotech GmbH) adjusted with a 0.5 mol/L methylene chloride solution and 0.4 mL of diisopropylethylamine (manufactured by Tokyo Chemical Industry Co., Ltd.) were added to the resin swollen with methylene chloride (manufactured by FUJIFILM Wako Pure Chemical Corporation), followed by shaking for 2 hours. The reaction was followed by washing with methylene chloride and N-methyl-2-pyrrolidone (manufactured by FUJIFILM Wako Pure Chemical Corporation). Next, after condensation of 1-naphthylacetic acid (manufactured by FUJIFILM Wako Pure Chemical Corporation) and deprotection of the allyl group, N-α-(9-fluorenylmethoxycarbonyl)-S-trityl-L-cysteine (manufactured by Watanabe Chemical Industries, Ltd.) was condensed. Next, after deprotection of the Fmoc group, 2-thiophenecarboxylic acid (manufactured by FUJIFILM Wako Pure Chemical Corporation) was condensed, followed by washing with N-methyl-2-pyrrolidone.

After completion of the peptide synthesis, the resin was washed with dichloromethane (manufactured by FUJIFILM Wako Pure Chemical Corporation), and then the solvent was distilled off under reduced pressure. 2 mL of trifluoroacetic acid (TFA) (manufactured by FUJIFILM Wako Pure Chemical Corporation):triisopropylsilane (manufactured by Tokyo Chemical Industry Co., Ltd.):water (= 95:2.5:2.5) was added to cleave the peptide from the resin while simultaneously carrying out deprotection. After 2 hours, the resin was filtered off, and 12 mL of n-hexane (manufactured by FUJIFILM Wako Pure Chemical Corporation):methyl-t-butyl ether (manufactured by FUJIFILM Wako Pure Chemical Corporation) (= 1:1) was added to the filtrate to generate a solid. The solid was precipitated by centrifugation and then the supernatant was removed. The solid was washed with methyl-t-butyl ether (manufactured by FUJIFILM Wako Pure Chemical Corporation), and then the solvent was distilled off under reduced pressure. Next, the intermediate crude product was dissolved in 2 mL of dimethylformamide (manufactured by FUJIFILM Wako Pure Chemical Corporation) to which 32 mg (0.2 mmol) of 1,1'-carbonyldiimidazole (manufactured by FUJIFILM Wako Pure Chemical Corporation) was then added, followed by stirring for 2 hours. Next, 25 mg (0.5 mmol) of hydrazine monohydrate (manufactured by FUJIFILM Wako Pure Chemical Corporation) was added thereto, followed by stirring for 2 hours. Then, water was added to the reaction solution, followed by extraction with ethyl acetate (manufactured by FUJIFILM Wako Pure Chemical Corporation), and the organic layer was washed with water and saturated saline and then dried over anhydrous sodium sulfate (manufactured by FUJIFILM Wako Pure Chemical Corporation). After removing the anhydrous sodium sulfate by filtration, the filtrate was distilled off under vacuum. The resulting residue was purified by liquid chromatography and then the solvent was distilled off under reduced pressure, followed by freeze-drying to obtain a white solid.

(Synthesis of Compound 11)

Solid phase peptide synthesis was carried out using 2-chlorotrityl chloride resin (manufactured by Watanabe Chemical Industries, Ltd.) as a resin for solid phase synthesis. The resin was used in an amount equivalent to 0.05 mmol. 0.075 mmol of (9H-fluoren-9-yl)methyl N-(2-sulfanylethyl)carbamate (synthesized by the method described in the

literature, Tetrahedron Letters, 2005, vol. 46, # 43, p. 7443) adjusted with a 0.5 mol/L methylene chloride solution and 0.4 mL of diisopropylethylamine (manufactured by Tokyo Chemical Industry Co., Ltd.) were added to the resin swollen with methylene chloride (manufactured by FUJIFILM Wako Pure Chemical Corporation), followed by shaking for 2 hours. The reaction was followed by washing with methylene chloride and N-methyl-2-pyrrolidone (manufactured by FUJIFILM Wako Pure Chemical Corporation). Next, N-α-(9-fluorenylmethoxycarbonyl)-L-aspartic acid β-allyl ester (manufactured by Watanabe Chemical Industries, Ltd.) and 1-naphthylacetic acid (manufactured by FUJIFILM Wako Pure Chemical Corporation) were condensed. After deprotection of the allyl group, hydrazine monohydrate (manufactured by FUJIFILM Wako Pure Chemical Corporation) was condensed.

After completion of the peptide synthesis, the resin was washed with dichloromethane (manufactured by FUJIFILM Wako Pure Chemical Corporation), and then the solvent was distilled off under reduced pressure. 2 mL of trifluoroacetic acid (TFA) (manufactured by FUJIFILM Wako Pure Chemical Corporation):triisopropylsilane (manufactured by Tokyo Chemical Industry Co., Ltd.):water (= 95:2.5:2.5) was added to cleave the peptide from the resin while simultaneously carrying out deprotection. After 2 hours, the resin was filtered off, and 12 mL of n-hexane (manufactured by FUJIFILM Wako Pure Chemical Corporation):methyl-t-butyl ether (manufactured by FUJIFILM Wako Pure Chemical Corporation) (= 1:1) was added to the filtrate to generate a solid. The solid was precipitated by centrifugation and then the supernatant was removed. The solid was washed with methyl-t-butyl ether (manufactured by FUJIFILM Wako Pure Chemical Corporation), and then the solvent was distilled off under reduced pressure. The resulting residue was purified by liquid chromatography and then the solvent was distilled off under reduced pressure, followed by freeze-drying to obtain a white solid.

(Synthesis of Compound 12)

Compound 12 was synthesized according to the synthesis method of Compound 11, except that (9H-fluoren-9-yl)methyl (R)-2-(mercaptomethyl)pyrrolidine-1-carboxylate (synthesized by the method described in the literature, Synlett, 2010, # 7, p. 1037) was used instead of (9H-fluoren-9-yl)methyl N-(2-sulfanylethyl)carbamate used in the synthesis of Compound 11.

(Synthesis of Compound 13)

120 mg (0.6 mmol) of 7-chloro-4-nitrobenzo-2-oxa-1,3-diazole (manufactured by Tokyo Chemical Industry Co., Ltd.) and 10 mL of dimethylformamide (manufactured by FUJIFILM Wako Pure Chemical Corporation) were placed and dissolved in an eggplant flask to which 200 mg (0.55 mmol) of S-trityl-L-cysteine (Cys(Trt)-OH) (manufactured by Tokyo Chemical Industry Co., Ltd.) and 250 μL of N,N-diisopropylethylamine (manufactured by FUJIFILM Wako Pure Chemical Corporation) were then added, followed by stirring for 2 hours. After completion of the reaction, water was added to the reaction solution, followed by extraction with ethyl acetate (manufactured by FUJIFILM Wako Pure Chemical Corporation), and the organic layer was washed with water and saturated saline and then dried over anhydrous sodium sulfate (manufactured by FUJIFILM Wako Pure Chemical Corporation).

Next, the entire amount of the intermediate crude product and 5 mL of dimethylformamide (manufactured by FUJIFILM Wako Pure Chemical Corporation) were placed and dissolved in an eggplant flask to which 113 mg (0.7 mmol) of 1,1'-carbonyldiimidazole (manufactured by FUJIFILM Wako Pure Chemical Corporation) was then added, followed by stirring for 2 hours. Next, 50 mg (1.0 mmol) of hydrazine monohydrate (manufactured by FUJIFILM Wako Pure Chemical Corporation) was added thereto, followed by stirring for 2 hours. Then, water was added to the reaction solution, followed by extraction with ethyl acetate (manufactured by FUJIFILM Wako Pure Chemical Corporation), and the organic layer was washed with water and saturated saline and then dried over anhydrous sodium sulfate (manufactured by FUJIFILM Wako Pure Chemical Corporation). After removing the anhydrous sodium sulfate by filtration, the filtrate was distilled off under vacuum.

Next, 2 mL of trifluoroacetic acid (TFA) (manufactured by FUJIFILM Wako Pure Chemical Corporation):triisopropylsilane (manufactured by Tokyo Chemical Industry Co., Ltd.):water (= 95:2.5:2.5) was added to the distillate. After stirring for 2 hours, the solvent was distilled off under reduced pressure. The resulting residue was purified by liquid chromatography and then the solvent was distilled off under reduced pressure, followed by freeze-drying to obtain a white solid.

(Synthesis of Compound 14)

Solid phase peptide synthesis was carried out using 2-chlorotrityl chloride resin (manufactured by Watanabe Chemical Industries, Ltd.) as a resin for solid phase synthesis. The resin was used in an amount equivalent to 0.05 mmol. 0.075 mmol of N-α-(9-fluorenylmethoxycarbonyl)-L-aspartic acid β-allyl ester (manufactured by Watanabe Chemical Industries, Ltd.) adjusted with a 0.5 mol/L methylene chloride solution and 0.4 mL of diisopropylethylamine (manufactured by Tokyo Chemical Industry Co., Ltd.) were added to the resin swollen with methylene chloride (manufactured by FUJIFILM Wako Pure Chemical Corporation), followed by shaking for 2 hours. The reaction was followed by washing with methylene chloride and N-methyl-2-pyrrolidone (manufactured by FUJIFILM Wako Pure Chemical Corporation). Next, after condensation of 2-[(triphenylmethyl)sulfanyl]ethanamine (manufactured by Combi-Blocks Inc.) and deprotection of the allyl group, mono-Fmoc ethylene diamine hydrochloride was condensed. Next, after deprotection of the Fmoc group, 2 mL of N-methyl-2-pyrrolidone solution of 12 mg (0.06 mmol) of 7-chloro-4-nitrobenzo-2-oxa-1,3-diazole (manufactured by Tokyo Chemical Industry Co., Ltd.) was added thereto, followed by shaking for 1 hour and then washing with N-methyl-2-pyrrolidone.

After completion of the peptide synthesis, the resin was washed with dichloromethane (manufactured by FUJIFILM Wako Pure Chemical Corporation), and then the solvent was distilled off under reduced pressure. 2 mL of trifluoroacetic acid (TFA) (manufactured by FUJIFILM Wako Pure Chemical Corporation):triisopropylsilane (manufactured by Tokyo Chemical Industry Co., Ltd.):water (= 95:2.5:2.5) was added to cleave the peptide from the resin while simultaneously carrying out deprotection. After 2 hours, the resin was filtered off, and 12 mL of n-hexane (manufactured by FUJIFILM Wako Pure Chemical Corporation):methyl-t-butyl ether (manufactured by FUJIFILM Wako Pure Chemical Corporation) (= 1:1) was added to the filtrate to generate a solid. The solid was precipitated by centrifugation and then the supernatant was removed. The solid was washed with methyl-t-butyl ether (manufactured by FUJIFILM Wako Pure Chemical Corporation), and then the solvent was distilled off under reduced pressure. Next, the intermediate crude product was dissolved in 2 mL of dimethylformamide (manufactured by FUJIFILM Wako Pure Chemical Corporation) to which 32 mg (0.2 mmol) of 1,1'-carbonyldiimidazole (manufactured by FUJIFILM Wako Pure Chemical Corporation) was then added, followed by stirring for 2 hours. Next, 25 mg (0.5 mmol) of hydrazine monohydrate (manufactured by FUJIFILM Wako Pure Chemical Corporation) was added thereto, followed by stirring for 2 hours. Then, water was added to the reaction solution, followed by extraction with ethyl acetate (manufactured by FUJIFILM Wako Pure Chemical Corporation), and the organic layer was washed with water and saturated saline and then dried over anhydrous sodium sulfate (manufactured by FUJIFILM Wako Pure Chemical Corporation). After removing the anhydrous sodium sulfate by filtration, the filtrate was distilled off under vacuum. The resulting residue was purified by liquid chromatography and then the solvent was distilled off under reduced pressure, followed by freeze-drying to obtain a white solid.

(Synthesis of Compound 15)

300 mg of 2,6-naphthalendiacetic acid (manufactured by A1 Biochem Labs), 295 mg of N-hydroxysuccinimide (manufactured by Tokyo Chemical Industry Co., Ltd.), and 10 mL of dimethylformamide (manufactured by FUJIFILM Wako Pure Chemical Corporation) were placed in an eggplant flask. Next, 671 mg of 1-(3-dimethylaminopropyl)-3-ethylcarbodiimide hydrochloride (manufactured by Dojindo Laboratories) was added thereto, followed by stirring for 2 hours. After completion of the reaction, water was added to the reaction solution, followed by extraction with ethyl acetate (manufactured by FUJIFILM Wako Pure Chemical Corporation), and the organic layer was washed with water and saturated saline and then dried over anhydrous sodium sulfate (manufactured by FUJIFILM Wako Pure Chemical Corporation). The anhydrous sodium sulfate was filtered off, and the filtrate was distilled off under vacuum. Then, the residue was purified by silica gel chromatography (n-hexane (manufactured by FUJIFILM Wako Pure Chemical Corporation): ethyl acetate (manufactured by FUJIFILM Wako Pure Chemical Corporation) = 1:0 to 0.7:0.3) to obtain 480 mg of a white solid bis(1,5-dioxopyrrolidin-1-yl) 2,2'-(naphthalene-2,6-diyl)diacetate.

Observed MS (ESI m/z): 439.2 (M + H), RT (min): 1.21

Next, 56 mg (0.13 mmol) of bis(1,5-dioxopyrrolidin-1-yl) 2,2'-(naphthalene-2,6-diyl)diacetate, 23 mg (0.06 mmol) of S-trityl-L-cysteine amide (manufactured by Combi-Blocks Inc.), and 1 mL of dimethylformamide (manufactured by FUJIFILM Wako Pure Chemical Corporation) were placed in an eggplant flask which was then immersed in an oil bath at 65°C and stirred for 3 hours. Then, the reaction solution was cooled to room temperature, and 25 mg (0.5 mmol) of hydrazine monohydrate (manufactured by FUJIFILM Wako Pure Chemical Corporation) was added thereto, followed by stirring for 1 hour. After completion of the reaction, water was added to the reaction solution, followed by extraction with ethyl acetate (manufactured by FUJIFILM Wako Pure Chemical Corporation), and the organic layer was washed with water and saturated saline and then dried over anhydrous sodium sulfate (manufactured by FUJIFILM Wako Pure Chemical Corporation). The anhydrous sodium sulfate was filtered off, and the filtrate was distilled off under vacuum. Next, 1 mL of trifluoroacetic acid (TFA) (manufactured by FUJIFILM Wako Pure Chemical Corporation):triisopropylsilane (manufactured by Tokyo Chemical Industry Co., Ltd.):water (= 95:2.5:2.5) was added thereto, and after 2 hours, 6 mL of n-hexane (manufactured by FUJIFILM Wako Pure Chemical Corporation):methyl-t-butyl ether (manufactured by FUJIFILM Wako Pure Chemical Corporation) (= 1:1) was added to generate a solid. The solid was precipitated by centrifugation and then the supernatant was removed. The solid was washed with methyl-t-butyl ether (manufactured by FUJIFILM Wako Pure Chemical Corporation), and then the solvent was distilled off under reduced pressure. The resulting

residue was purified by liquid chromatography and then the solvent was distilled off under reduced pressure, followed by freeze-drying to obtain a white solid.

(Synthesis of Comparative Example 1)

Solid phase peptide synthesis was carried out using Rink Amide-ChemMatrix (manufactured by Biotage AB) (0.45 mmol/g) as a resin for solid phase synthesis. The resin was used in an amount of 111.1 mg (0.05 mmol).

Condensation was carried out in the order of (S)-2-((((9H-fluoren-9-yl)methoxy)carbonyl)amino)-S-trityl-L-cysteine (Fmoc-Cys(Trt)-OH) (manufactured by Watanabe Chemical Industries, Ltd.) and 1-naphthylacetic acid (manufactured by FUJIFILM Wako Pure Chemical Corporation). After completion of the elongation, the resin was washed with dichloromethane (manufactured by FUJIFILM Wako Pure Chemical Corporation), and then the solvent was distilled off under reduced pressure. 2 mL of trifluoroacetic acid (TFA) (manufactured by FUJIFILM Wako Pure Chemical Corporation):triisopropylsilane (manufactured by Tokyo Chemical Industry Co., Ltd.):water (= 95:2.5:2.5) was added to cleave the peptide from the resin while simultaneously carrying out deprotection. After 2 hours, the resin was filtered off, and 12 mL of n-hexane (manufactured by FUJIFILM Wako Pure Chemical Corporation):methyl-t-butyl ether (manufactured by FUJIFILM Wako Pure Chemical Corporation) (= 1:1) was added to the filtrate to generate a solid. The solid was precipitated by centrifugation and then the supernatant was removed. The solid was washed with methyl-t-butyl ether (manufactured by FUJIFILM Wako Pure Chemical Corporation), and then the solvent was distilled off under reduced pressure. The resulting residue was purified by liquid chromatography and then the solvent was distilled off under reduced pressure, followed by freeze-drying to obtain 6.4 mg of a white solid.
Observed MS (ESI m/z): 289.2 (M + H), RT (min): 1.09

Solid phase peptide synthesis method using automatic peptide synthesizer

Solid phase peptide synthesis was carried out using an automatic peptide synthesizer (Syrol, manufactured by Biotage AB). Synthesis was carried out by setting a resin for solid phase synthesis, N-methyl-2-pyrrolidone (NMP) solutions of 4 equivalents of Fmoc amino acids (0.5 mol/L) with respect to the resin, an NMP solution of 4 equivalents of ethyl cyanohydroxyiminoacetate (1 mol/L) with respect to the resin, an NMP solution of 4 equivalents of diisopropylcarbodiimide (1 mol/L) with respect to the resin, and an NMP solution of piperidine (20% v/v) in the synthesizer. A cycle of Fmoc deprotection (20 minutes), washing with NMP, condensation of Fmoc amino acids (1 hour), and washing with NMP was repeated to elongate the peptide chain.

In the deprotection of the allyl group, 58 mg (0.05 mmol) of tetrakis(triphenylphosphine)palladium (0) (manufactured by Tokyo Chemical Industry Co., Ltd.), 1.85 mL of chloroform (manufactured by FUJIFILM Wako Pure Chemical Corporation), 0.1 mL of acetic acid (manufactured by FUJIFILM Wako Pure Chemical Corporation), and 0.05 mL of N-methylmorpholine (manufactured by FUJIFILM Wako Pure Chemical Corporation) were added, followed by shaking for 2 hours. The completion of the reaction was followed by washing with NMP.

Purification of the obtained crude product was carried out by liquid chromatography.

Column: XSelect CSH Prep C18 5 μm OBD (19 × 250 mm), manufactured by Waters Corporation
Column temperature: 40°C
Flow rate: 20 mL/min
Detection wavelength: 220 nm, 254 nm
Solvent: liquid A: 0.1% formic acid-water

liquid B: 0.1% formic acid-acetonitrile
Fmoc amino acids were obtained from Watanabe Chemical Industries, Ltd.
N-methyl-2-pyrrolidone, diisopropylethylamine, diisopropylcarbodiimide, piperidine, and anhydrous acetic acid were obtained from FUJIFILM Wako Pure Chemical Corporation. Ethyl cyanohydroxyiminoacetate was obtained from Tokyo Chemical Industry Co., Ltd.

The mass spectrum (MS) was measured using an ACQUITY SQD LC/MS System (manufactured by Waters Corporation, ionization method: electrospray ionization (ESI) method).

The retention time (RT) was measured using an ACQUITY SQD LC/MS System (manufactured by Waters Corporation) and indicated in minutes (min).

Column: BEHC 18 1.7 μm, 2.1 × 30 mm (manufactured by Waters Corporation)
Solvent: liquid A: 0.1% formic acid-water
liquid B: 0.1% formic acid-acetonitrile

Gradient cycle: 0.00 min (liquid A/liquid B = 95/5), 2.00 min (liquid A/liquid B = 5/95), 3.00 min (liquid A/liquid B = 95/5)
Flow rate: 0.5 mL/min
Column temperature: room temperature
Detection wavelength: 254 nm

(Synthesis of Comparative Example 2)

Synthesis was carried out with reference to Example 1 to Example 5 of JP2009-222466A.

<Test method>

(1) Preparation of various solutions

(1-1) 0.1 mmol/L EDTA aqueous solution

1) 37.2 mg of EDTA·2Na·$2H_2O$ (manufactured by Dojindo Laboratories) is weighed into a 15 mL conical tube, and 10 mL of distilled water (manufactured by Hikari Pharmaceutical Co., Ltd., water for injection according to the Japanese Pharmacopoeia) is added using a 25 mL measuring pipette to dissolve EDTA·2Na·$2H_2O$ (10 mmol/L EDTA aqueous solution).
2) 49.5 mL of distilled water (manufactured by Hikari Pharmaceutical Co., Ltd., water for injection according to the Japanese Pharmacopoeia) is added to a 100 mL container using a 50 mL measuring pipette, and 0.5 mL of the 10 mmol/L EDTA aqueous solution of 1) is added thereto, followed by mixing to dilute the 10 mmol/L EDTA aqueous solution 100-fold (0.1 mmol/L EDTA aqueous solution).

(1-2) 100 mmol/L phosphate buffer solution (pH 7.4 and pH 8.0)

1) 0.6 g of anhydrous sodium dihydrogen phosphate (manufactured by FUJIFILM Wako Pure Chemical Corporation, special grade) is weighed into a 100 mL container, and 50 mL of distilled water (manufactured by Hikari Pharmaceutical Co., Ltd., water for injection according to the Japanese Pharmacopoeia) is added using a 50 mL measuring pipette to dissolve the anhydrous sodium dihydrogen phosphate.
2) 300 mL of distilled water (manufactured by Hikari Pharmaceutical Co., Ltd., water for injection according to the Japanese Pharmacopoeia) is added to a 500 mL container using a 50 mL (or 100 mL) measuring pipette.
3) 4.26 g of anhydrous disodium hydrogen phosphate (manufactured by FUJIFILM Wako Pure Chemical Corporation, special grade) is weighed and is added and dissolved in the distilled water of 2) (manufactured by Hikari Pharmaceutical Co., Ltd., water for injection according to the Japanese Pharmacopoeia).
4) While measuring the pH with a pH meter, an appropriate amount of the anhydrous sodium dihydrogen phosphate solution of 1) is added to the anhydrous disodium hydrogen phosphate solution of 3) using a 25 mL measuring pipette to adjust the pH to 7.4 or 8.0.
5) a volume of 299 mL from the solution of 4) is transferred to a new 500 mL container using a 50 mL measuring pipette, and 1 mL of a 0.1 mmol/L EDTA aqueous solution is added thereto to make a volume of 300 mL. The concentration of EDTA in this solution is 0.33 μmol/L, and the concentration of EDTA in the reaction solution is 0.25 μmol/L.
6) The above solution is filtered and sterilized through a 0.22 μm filter.

(1-3) Reaction stop solution

1) Reaction stop solution for UV detection (2.5% (v/v) TFA aqueous solution)

2.5 mL of TFA (manufactured by FUJIFILM Wako Pure Chemical Corporation, special grade) is added to 100 mL of distilled water (manufactured by FUJIFILM Wako Pure Chemical Corporation).

2) Reaction stop solution for fluorescence detection (0.5% (v/v) TFA aqueous solution)

0.5 mL of TFA (manufactured by FUJIFILM Wako Pure Chemical Corporation, special grade) is added to 100

mL of distilled water (manufactured by FUJIFILM Wako Pure Chemical Corporation).

(1-4) HPLC mobile phase A: 0.1% (v/v) TFA aqueous solution

1.0 mL of TFA is added to 1 L of distilled water (manufactured by FUJIFILM Wako Pure Chemical Corporation).

(1-5) HPLC mobile phase B: 0.1% (v/v) TFA acetonitrile solution

1.0 mL of TFA is added to 1 L of HPLC grade acetonitrile (manufactured by FUJIFILM Wako Pure Chemical Corporation, for HPLC).

(2) Preparation of nucleophilic reagent stock solution

The same stock solution is used for each test and stored in aliquots for single use. A specific preparation example of the nucleophilic reagent stock solution is shown below.

1) The nucleophilic reagent is dissolved in DMSO (manufactured by FUJIFILM Wako Pure Chemical Corporation) according to its molecular weight to prepare a 2 mmol/L nucleophilic reagent solution.
2) 149.5 mL of the same buffer solution is added to a 500 mL container using a 50 mL measuring pipette, and 0.5 mL of the 2 mmol/L nucleophilic reagent solution is added thereto, followed by mixing by inversion to dilute the 2 mmol/L nucleophilic reagent solution 300-fold (6.667 μmol/L). This solution is stored frozen at -70°C or lower.

(3) Preparation of test substance solution

One type of solvent for which a 1 mmol/L test substance solution can be prepared is selected according to the priority order of water, acetonitrile, acetone, and an acetonitrile solution of 5% DMSO. In a case where water, acetonitrile, or acetone is selected, first, a 20 mmol/L test substance solution is prepared. A solvent is added to the test substance weighed in an appropriate amount so as to obtain a 20 mmol/L solution, and the test substance is completely dissolved. Thereafter, a portion of the 20 mmol/L solution is taken and diluted 20-fold with the same solvent to prepare a 1 mmol/L test substance solution. In a case where an acetonitrile solution of 5% by mass DMSO is selected, a 20 mmol/L DMSO solution is prepared in the same manner as described above. Thereafter, a portion of the 20 mmol/L DMSO solution is taken and diluted 20-fold with acetonitrile to prepare a 1 mmol/L test substance solution.

(4) Reaction

(4-1) Addition

The test substance solution is prepared on a 96-well plate (U96 PP-0.5 ML NATURAL, manufactured by Thermo Fisher Scientific (NUNC) Inc.) mainly using a 12-channel pipette, and the reagent is added according to the following doses.

Nucleophilic reagent: 150 μL
Test substance solution: 50 μL

(4-2) Reaction

The plate is tightly sealed with a plate seal (TORAST™ 96well Seal E Type, manufactured by Shimadzu GLC Ltd.), and stirred with a plate shaker (Titramax 100, manufactured by Heidolph Instruments GmbH & CO. KG). After spinning down in a centrifuge, the plate is incubated at 25°C for 24 hours in a light-shielded state.

(4-3) Stop of reaction

After incubation for 24 hours, the plate seal is peeled off, and in the HPLC measurement which will be described later, 50 μL of a reaction stop solution for UV detection (2.5% (v/v) TFA aqueous solution) is added to each sample in a case of measurement by UV detection to stop the reaction. In a case of measurement by fluorescence detection, 180 μL of a reaction stop solution for fluorescence detection (0.5% (v/v) TFA aqueous solution) is dispensed into a new plate, and 20 μL of the reaction solution after incubation is added thereto to stop the reaction.

(5) HPLC measurement

The HPLC measurement conditions of the nucleophilic reagent are shown below. As for the elution condition, Condition 1, Condition 2, or Condition 3 was selected depending on the nucleophilic reagent.

[Table 1]

<table>
<tr><td>HPLC equipment</td><td colspan="4">LC-20A (Prominence) series (Shimadzu Corporation)</td></tr>
<tr><td>Column</td><td colspan="4">Wakopak (registered trademark) Core C18 ADRA column (3.0 x 150 mm, 2.6 μm) (FUJIFILM Wako Pure Chemical Corporation) or SunShell Phenyl (3.0 × 150 mm, 2.6 μm) (Chromanik Technologies Inc.)</td></tr>
<tr><td rowspan="2">Detector</td><td colspan="4">UV detection: SPD-M20A (Shimadzu Corporation)</td></tr>
<tr><td colspan="4">Fluorescence detection: RF 20AXS (Shimadzu Corporation)</td></tr>
<tr><td rowspan="2">Detection wavelength</td><td colspan="4">UV detection: 281 nm</td></tr>
<tr><td colspan="4">Fluorescence detection: 284 nm (excitation), 333 nm (fluorescence)</td></tr>
<tr><td>Column temperature</td><td colspan="4">40°C</td></tr>
<tr><td>Sample temperature</td><td colspan="4">4°C or 25°C</td></tr>
<tr><td>Injection volume</td><td colspan="4">10 to 20 μL</td></tr>
<tr><td rowspan="2">Eluent</td><td colspan="4">A: water (0.1 % trifluoroacetic acid)</td></tr>
<tr><td colspan="4">B: acetonitrile (0.1% trifluoroacetic acid)</td></tr>
<tr><td>Measurement time</td><td colspan="4">20 min</td></tr>
<tr><td rowspan="24">Elution condition</td><td colspan="4">Condition 1</td></tr>
<tr><td>Time (min)</td><td>Flow rate (ml/min)</td><td>%A</td><td>%B</td></tr>
<tr><td>0.0</td><td>0.3</td><td>70</td><td>30</td></tr>
<tr><td>9.5</td><td>0.3</td><td>45</td><td>55</td></tr>
<tr><td>10.0</td><td>0.3</td><td>0</td><td>100</td></tr>
<tr><td>13.0</td><td>0.3</td><td>0</td><td>100</td></tr>
<tr><td>13.5</td><td>0.3</td><td>70</td><td>30</td></tr>
<tr><td>20.0</td><td>End</td><td></td><td></td></tr>
<tr><td colspan="4">Condition 2</td></tr>
<tr><td>Time (min)</td><td>Flow rate (ml/min)</td><td>%A</td><td>%B</td></tr>
<tr><td>0.0</td><td>0.3</td><td>80</td><td>20</td></tr>
<tr><td>9.5</td><td>0.3</td><td>55</td><td>45</td></tr>
<tr><td>10.0</td><td>0.3</td><td>0</td><td>100</td></tr>
<tr><td>13.0</td><td>0.3</td><td>0</td><td>100</td></tr>
<tr><td>13.5</td><td>0.3</td><td>80</td><td>20</td></tr>
<tr><td>20.0</td><td>End</td><td></td><td></td></tr>
<tr><td colspan="4">Condition 3</td></tr>
<tr><td>Time (min)</td><td>Flow rate (ml/min)</td><td>%A</td><td>%B</td></tr>
<tr><td>0.0</td><td>0.3</td><td>75</td><td>25</td></tr>
<tr><td>9.5</td><td>0.3</td><td>50</td><td>50</td></tr>
<tr><td>10.0</td><td>0.3</td><td>0</td><td>100</td></tr>
<tr><td>13.0</td><td>0.3</td><td>0</td><td>100</td></tr>
<tr><td>13.5</td><td>0.3</td><td>75</td><td>25</td></tr>
<tr><td>20.0</td><td>End</td><td></td><td></td></tr>
</table>

(6) Data analysis

(6-1) Calculation of % depletion

% depletion of the nucleophilic reagent is calculated according to the following equation from the average value of the peak areas of the nucleophilic reagent.

% depletion of nucleophilic reagent = [1 - (average value of peak areas of unreacted nucleophilic reagent after reaction/average value of peak areas of standard nucleophilic reagent)] × 100

(7) Evaluation items

(7-1) Stability of nucleophilic reagent (in particular, degree of oxidation of cysteine)

Immediately after preparation of the reaction solution (0 hours) and after incubation of the reaction solution at 25°C for 24 hours (24 hours), the reaction solution is measured by HPLC-UV. At this time, since the nucleophilic reagent and the oxidized form and modified form of the nucleophilic reagent can be confirmed on HPLC, the residual ratio of the nucleophilic reagent is calculated based on the following equation.

Residual ratio (%) of nucleophilic reagent = area value of nucleophilic reagent/(area value of nucleophilic reagent + area value of oxidized form of nucleophilic reagent + area value of modified form of nucleophilic reagent) × 100

(7-2) Fluorescence detection sensitivity of nucleophilic reagent

The fluorescence detection determines the fluorescence of the naphthalene ring (peak area detected at an excitation wavelength of 284 nm and a fluorescence wavelength of 333 nm).

(7-3) Evaluation of reactivity with sensitizing substance

Fifteen substances shown in the table below were used for the evaluation of the reactivity. These fifteen substances were selected to include substances that are difficult to distinguish between sensitization and non-sensitization in the related art DPRA and ADRA.

[Table 2]

| No. | Evaluation substance | CAS | Molecular weight | Purity (%) | Skin sensitization | Solvent used |
|---|---|---|---|---|---|---|
| 1 | Diphenylcyclopropenone (FUJIFILM Wako Pure Chemical Corporation) | 886-38-4 | 206.24 | 98 | Particularly strong sensitizing substance | Acetonitrile |
| 2 | Trimellitic anhydride (FUJIFILM Wako Pure Chemical Corporation) | 552-30-7 | 192.13 | 97 | Strong sensitizing substance | Acetonitrile |
| 3 | Nonanoyl chloride (Tokyo Chemical Industry Co., Ltd.) | 764-85-2 | 176.68 | 97 | Moderate sensitizing substance | Acetonitrile |
| 4 | Methyl pyruvate (FUJIFILM Wako Pure Chemical Corporation) | 600-22-6 | 102.09 | 95 | Moderate sensitizing substance | Water |

(continued)

| No. | Evaluation substance | CAS | Molecular weight | Purity (%) | Skin sensitization | Solvent used |
|---|---|---|---|---|---|---|
| 5 | Diethyl sulfate (Tokyo Chemical Industry Co., Ltd.) | 64-67-5 | 154.18 | 98 | Moderate sensitizing substance | Water |
| 6 | 3-Propylidene phthalide (Tokyo Chemical Industry Co., Ltd.) | 17369-59-4 | 174.20 | 96 | Moderate sensitizing substance | Acetonitrile |
| 7 | Tropolone (FUJIFILM Wako Pure Chemical Corporation) | 533-75-5 | 122.12 | 97 | Moderate sensitizing substance | Water |
| 8 | 10-Undecenal (FUJIFILM Wako Pure Chemical Corporation) | 112-45-8 | 168.28 | 95 | Moderate sensitizing substance | Acetonitrile |
| 9 | $\alpha$-Pentyl cinnamaldehyde (FUJIFILM Wako Pure Chemical Corporation) | 122-40-7 | 202.29 | 98 | Weak sensitizing substance | Acetonitrile |
| 10 | Phenyl benzoate (Tokyo Chemical Industry Co., Ltd.) | 93-99-2 | 198.22 | 99 | Weak sensitizing substance | Acetonitrile |
| 11 | Cyclamen aldehyde (Sigma-Aldrich Co. LLC.) | 103-95-7 | 190.28 | 92 | Weak sensitizing substance | Acetonitrile |
| 12 | 1-Bromobutane (FUJIFILM Wako Pure Chemical Corporation) | 109-65-9 | 137.02 | 95 | Non-sensitizing substance | Acetonitrile |
| 13 | 1-Iodohexane (Sigma-Aldrich Co. LLC.) | 638-45-9 | 212.07 | 98 | Non-sensitizing substance | Acetonitrile |
| 14 | 4'-Methoxyacetophenone (Tokyo Chemical Industry Co., Ltd.) | 100-06-1 | 150.18 | 99 | Non-sensitizing substance | Acetonitrile |
| 15 | Ethyl benzoylacetate (FUJIFILM Wako Pure Chemical Corporation) | 94-02-0 | 192.21 | 90 | Non-sensitizing substance | Acetonitrile |

The reactivity was evaluated by comparison with % depletion of cysteine peptides and lysine peptides in the related art DPRA, % depletion of NAC and NAL in ADRA, % depletion of NAC having an amide N-terminal (NAC-amide), and % depletion of the reagent (GSH-NBD) described in JP2009-222466A.

<Example 1>

One type of nucleophilic reagent (Compound 1) was evaluated. In addition, the following compounds were also evaluated in the same manner as controls for comparison of the reactivity to the fifteen types of evaluation substances.

Cys peptide

Lys peptide

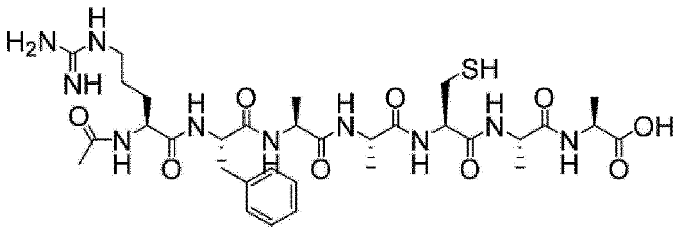

Cys peptide

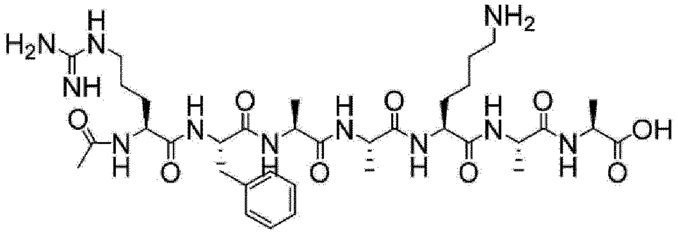

Lys peptide

NAC: N-[2-(naphthalen-1-yl)acetyl]cysteine
NAL: α-N-[2-(naphthalen-1-yl)acetyl]lysine
NAC-amide: (R)-3-mercapto-2-(2-(naphthalen-1-yl)acetamide)propanamide
GSH-NBD: compound described in paragraph [0043] of JP2009-222466A

(Test substance and solution preparation)

A 1 mmol/L solution was prepared for each of the fifteen substances shown in the foregoing section of "(7-3) Evaluation of reactivity with sensitizing substance" and used in the test. For Compound 1, a stock solution prepared by using a buffer solution having a pH of 7.4 or a pH of 8.0 in a case of preparing a 6.667 μmol/L solution in the foregoing section of "(2) Preparation of nucleophilic reagent stock solution" was used. For NAC-amide and GSH-NBD, only a stock solution prepared by using a buffer solution having a pH of 8.0 was used.

(Measurement conditions)

The depletion (%) of the nucleophilic reagent was determined according to the HPLC measurement conditions described in the foregoing section of "(5) HPLC measurement". In this regard, in GSH-NBD, detection in HPLC was carried out by UV detection at a detection wavelength of 338 nm.

(Results)

(1) Stability of nucleophilic reagent

With regard to Compound 1, the residual ratios immediately after solution preparation (0 hours) and after 24 hours were calculated. The results are shown in Fig. 1. Compound 1 remained at 90% or more at 0 hours and remained at 85% or more even after 24 hours, showing no significant decrease in residual ratio.

(2) Fluorescence detection sensitivity of nucleophilic reagent

For the nucleophilic reagent for which a stock solution was prepared using a buffer solution having a pH of 8.0, the fluorescence intensity (peak area in HPLC) was measured immediately after solution preparation (0 hours). The results are shown in Fig. 2. A peak area sufficient to quantify the nucleophilic reagent was detected.

(3) Reactivity of nucleophilic reagent

For Compound 1, the reactivity with respect to the fifteen types of evaluation substances was calculated. Fig.

3 shows the results of comparison with the depletion of each nucleophilic reagent in substances No. 1 to No. 8 described in Table 2 of the foregoing section of "(7-3) Evaluation of reactivity with sensitizing substance", and Fig. 4 shows the results of comparison with the depletion of each nucleophilic reagent in substances No. 9 to No. 15.

As a result, first, in a case of comparison with the cysteine peptide (Cys peptide) and the lysine peptide (Lys peptide) in DPRA, Compound 1 showed higher reactivity than the cysteine peptide and the lysine peptide with respect to five types of sensitizing substances, nonanoyl chloride, methyl pyruvate, 10-undecenal, α-pentyl cinnamaldehyde, and cyclamen aldehyde. On the other hand, Compound 1 showed lower reactivity with respect to four types of sensitizing substances, diethyl sulfate, 3-propylidene phthalide, tropolone, and phenyl benzoate, compared to the cysteine peptide or the lysine peptide, but had a depletion of 5% or more for all of these four sensitizing substances, confirming a certain degree of reactivity. In addition, Compound 1 showed no reactivity with respect to 1-bromobutane and 1-iodohexane, which are non-sensitizing substances but are reactive with the cysteine peptide in DPRA. The results were consistent with the actual sensitization information (non-sensitization). Compound 1 showed the same level of reactivity as the cysteine peptide or the lysine peptide with respect to four substances (diphenylcyclopropenone, trimellitic anhydride, 4'-methoxyacetophenone, and ethyl benzoylacetate) other than the above-mentioned substances.

Next, in a case of comparison with NAC and NAL in ADRA, Compound 1 showed higher reactivity than NAC and NAL with respect to nine types of sensitizing substances, diphenylcyclopropenone, nonanoyl chloride, methyl pyruvate, diethyl sulfate, tropolone, 10-undecenal, α-pentyl cinnamaldehyde, phenyl benzoate, and cyclamen aldehyde. On the other hand, NAL showed higher reactivity than Compound 1 with respect to trimellitic anhydride, but the depletion of Compound 1 was 41.0% (pH 7.4) and 37.2% (pH 8.0), confirming sufficient reactivity. Compound 1 showed the same level of reactivity as NAC or NAL with respect to five substances (3-propylidene phthalide, 1-bromobutane, 1-iodohexane, 4'-methoxyacetophenone, and ethyl benzoylacetate) other than the above-mentioned substances.

In a case of comparison with NAC-amide, Compound 1 showed higher reactivity with respect to five types of sensitizing substances, trimellitic anhydride, methyl pyruvate, 10-undecenal, α-pentyl cinnamaldehyde, and cyclamen aldehyde. Compound 1 showed the same level of reactivity as NAC-amide with respect to ten types of substances (diphenylcyclopropenone, nonanoyl chloride, diethyl sulfate, 3-propylidene phthalide, tropolone, phenyl benzoate, 1-bromobutane, 1-iodohexane, 4'-methoxyacetophenone, and ethyl benzoylacetate) other than the above-mentioned substances.

In a case of comparison with GSH-NBD, Compound 1 showed higher reactivity with respect to five types of sensitizing substances, methyl pyruvate, diethyl sulfate, 10-undecenal, α -pentyl cinnamaldehyde, and cyclamen aldehyde. On the other hand, GSH-NBD showed higher reactivity with respect to trimellitic anhydride and nonanoyl chloride, but the depletion of Compound 1 was 35% or more for both substances, confirming sufficient reactivity. Compound 1 and GSH-NBD showed similar reactivity with respect to eight types of substances (diphenylcyclopropenone, 3-propylidene phthalide, tropolone, phenyl benzoate, 1-bromobutane, 1-iodohexane, 4'-methoxyacetophenone, and ethyl benzoylacetate) other than the above-mentioned substances.

Regarding the reactivity (depletion) of Compound 1, NAC-amide, and GSH-NBD with respect to fifteen types of evaluation substances, prediction of skin sensitization was carried out using, as a determination criterion, the depletion of 5.6%, which is a determination condition in the method of predicting skin sensitization with NAC alone in the related art ADRA. Regarding the results of Compound 1, Table 3 shows the comparison of the prediction results of NAC-amide and GSH-NBD carried out above with the prediction results of DPRA and ADRA published in the literature.

[Table 3]

| No. | Compound name | Category of LLNA sensitization | DPRA prediction | ADRA prediction | Comparative Example 1 prediction | Comparative Example 2 prediction | Compound 1 prediction | |
|---|---|---|---|---|---|---|---|---|
| | | | | | | | pH 7.4 | pH 8.0 |
| 1 | Diphenylcyclopropenone | Particularly strong | Positive | Positive | Positive | Positive | Positive | Positive |
| 2 | Trimellitic anhydride | Strong | Positive | Positive | Falsely negative | Positive | Positive | Positive |
| 3 | Nonanoyl chloride | Moderate | Falsely negative | Positive | Positive | Positive | Positive | Positive |
| 4 | Methyl pyruvate | Moderate | Falsely negative | Falsely negative | Falsely negative | Falsely negative | Positive | Positive |
| 5 | Diethyl sulfate | Moderate | Positive | Falsely negative | Positive | Falsely negative | Positive | Positive |
| 6 | 3-Propylidene phthalide | Moderate | Positive | Falsely negative | Falsely negative | Falsely negative | Positive | Positive |
| 7 | Tropolone | Moderate | Positive | Falsely negative | Positive | Positive | Positive | Positive |
| 8 | 10-Undecenal | Moderate | Falsely negative | Falsely negative | Falsely negative | Positive | Positive | Positive |
| 9 | α-Pentyl cinnamaldehyde | Weak | Falsely negative | Falsely negative | Falsely negative | Falsely negative | Positive | Positive |
| 10 | Phenyl benzoate | Weak | Positive | Falsely negative | Positive | Positive | Positive | Positive |
| 11 | Cyclamen aldehyde | Weak | Positive | Falsely negative | Positive | Positive | Positive | Positive |
| 12 | 1-Bromobutane | None | Falsely positive | Negative | Negative | Negative | Negative | Negative |
| 13 | 1-Iodohexane | None | Falsely positive | Negative | Negative | Negative | Negative | Negative |
| 14 | 4'-Methoxyacetophenone | None | Negative | Negative | Negative | Negative | Negative | Negative |
| 15 | Ethyl benzoylacetate | None | Negative | Negative | Negative | Negative | Negative | Negative |

As a result of the above, four types of sensitizing substances (nonanoyl chloride, methyl pyruvate, 10-undecenal, and α-pentyl cinnamaldehyde) that were erroneously determined to be negative by DPRA were correctly determined to be positive by Compound 1. Similarly, eight types of sensitizing substances (methyl pyruvate, diethyl sulfate, 3-propylidene phthalide, tropolone, 10-undecenal, α-pentyl cinnamaldehyde, phenyl benzoate, and cyclamen aldehyde) that were erroneously determined to be negative by ADRA were correctly determined to be positive by Compound 1. With regard to NAC-amide, five types of sensitizing substances (trimellitic anhydride, methyl pyruvate, 3-propylidene phthalide, 10-undecenal, and α-pentyl cinnamaldehyde) that were erroneously determined to be negative were correctly determined to be positive by Compound 1. With regard to GSH-NBD, four types of sensitizing substances (methyl pyruvate, diethyl sulfate, 3-propylidene phthalide, and α-pentyl cinnamaldehyde) that were erroneously determined to be negative were correctly determined to be positive by Compound 1. In addition, two types of non-sensitizing substances (1-bromobutane and 1-iodohexane) that were erroneously determined to be positive by DPRA were correctly determined to be negative by Compound 1.

From the above, it is considered that Compound 1 may be able to predict sensitizing substances with higher sensitivity than the related art DPRA and ADRA, which makes it possible to highly correctly evaluate sensitizing substances that were difficult to predict by conventional skin sensitization measurement methods.

## Claims

1. A reagent for measuring skin sensitization comprising, as a main measuring agent:
an organic compound having a mercapto group and a hydrazide structure and having an absorption spectrum in an ultraviolet, visible, or near-infrared region.

2. The reagent for measuring skin sensitization according to claim 1,
wherein the organic compound is represented by Formula (1) or Formula (2),

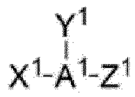
(1)

$X^2$-$Y^2$-$Z^2$ (2)

in the formulae,

$A^1$ represents a nitrogen atom or the following linking group,

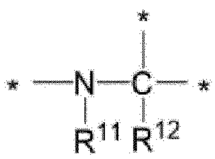
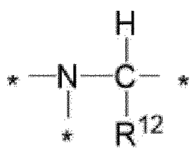
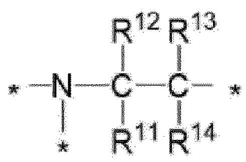
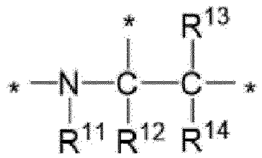
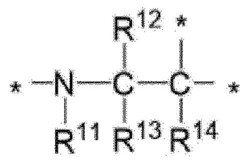

$R^{11}$, $R^{12}$, $R^{13}$, and $R^{14}$ each independently represent a hydrogen atom, an alkyl group having 1 to 10 carbon atoms, an alkenyl group having 2 to 10 carbon atoms, an alkynyl group having 2 to 10 carbon atoms, a cycloalkyl group having 3 to 10 carbon atoms, or a cycloalkenyl group having 3 to 10 carbon atoms, each of which may contain -O-, -C(O)-, -OC(O)-, -$NJ^1$-CO-, -CO-$NJ^1$-, or -NH-CO-NH- in a molecular chain, $J^1$ represents a hydrogen atom or an alkyl group having 1 to 3 carbon atoms, and the alkyl group, the alkenyl group, the alkynyl group, the cycloalkyl group, or the cycloalkenyl group may have a substituent selected from a cycloalkyl group having 3 to 6 carbon atoms, a cycloalkenyl group having 5 to 6 carbon atoms, an amino group, a cyano group, a

mercapto group, a mercaptomethyl group, a hydroxyl group, a phenyl group, a hydroxyphenyl group, a pyridyl group, a naphthyl group, a thienyl group, or a furyl group,
* represents a connection position with $X^1$, $Y^1$, or $Z^1$,
$X^1$ and $X^2$ represent an alkyl group having one or more mercapto groups and having 1 to 10 carbon atoms, an alkenyl group having one or more mercapto groups and having 2 to 10 carbon atoms, an alkynyl group having one or more mercapto groups and having 2 to 10 carbon atoms, a cycloalkyl group having one or more mercapto groups and having 3 to 10 carbon atoms, a cycloalkenyl group having one or more mercapto groups and having 3 to 10 carbon atoms, an arylalkyl group having one or more mercapto groups and having 7 to 12 carbon atoms, a heteroalkylalkyl group having one or more mercapto groups and having 3 to 10 carbon atoms, or a mercapto group, each of which may contain -O-, -C(O)-, -OC(O)-, $-NJ^2-CO-$, $-CO-NJ^2-$, or -NH-CO-NH- in a molecular chain, $J^2$ represents a hydrogen atom or an alkyl group having 1 to 3 carbon atoms, and the alkyl group, the alkenyl group, the alkynyl group, the cycloalkyl group, or the cycloalkenyl group may have a substituent selected from a cycloalkyl group having 3 to 6 carbon atoms, a cycloalkenyl group having 5 to 6 carbon atoms, an amino group, a cyano group, a mercaptomethyl group, a hydroxyl group, a phenyl group, a hydroxyphenyl group, a pyridyl group, a naphthyl group, a thienyl group, or a furyl group,
$Y^1$ and $Y^2$ represent a group having 6 to 20 carbon atoms and containing a structure having an absorption spectrum in an ultraviolet, visible, or near-infrared region, and
$Z^1$ and $Z^2$ represent $-CO-NR^{21}NR^{22}R^{23}$, where $R^{21}$, $R^{22}$, and $R^{23}$ each independently represent a hydrogen atom or an alkyl group having 1 to 10 carbon atoms.

**3.** The reagent for measuring skin sensitization according to claim 1, wherein the organic compound is represented by Formula (10),

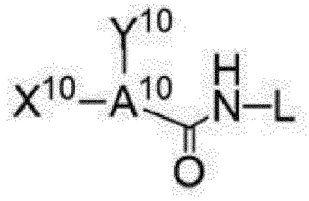

(10)

in the formula,

$A^{10}$ represents a nitrogen atom or a trivalent linking group,
$X^{10}$ represents an alkyl group having one or more mercapto groups and having 1 to 10 carbon atoms, an alkenyl group having one or more mercapto groups and having 2 to 10 carbon atoms, an alkynyl group having one or more mercapto groups and having 2 to 10 carbon atoms, a cycloalkyl group having one or more mercapto groups and having 3 to 10 carbon atoms, a cycloalkenyl group having one or more mercapto groups and having 3 to 10 carbon atoms, an arylalkyl group having one or more mercapto groups and having 7 to 12 carbon atoms, a heteroalkylalkyl group having one or more mercapto groups and having 3 to 10 carbon atoms, or a mercapto group, each of which may contain -O-, -C(O)-, -OC(O)-, $-NJ^{101}-CO-$, $-CO-NJ^{101}-$, or -NH-CO-NH- in a molecular chain, $J^{101}$ represents a hydrogen atom or an alkyl group having 1 to 3 carbon atoms, and the alkyl group, the alkenyl group, the alkynyl group, the cycloalkyl group, or the cycloalkenyl group may have a substituent selected from a cycloalkyl group having 3 to 6 carbon atoms, a cycloalkenyl group having 5 to 6 carbon atoms, an amino group, a cyano group, a mercaptomethyl group, a hydroxyl group, a phenyl group, a hydroxyphenyl group, a pyridyl group, a naphthyl group, a thienyl group, or a furyl group,
$Y^{10}$ represents a group having 6 to 20 carbon atoms and containing a structure having an absorption spectrum in an ultraviolet, visible, or near-infrared region, and
L represents an amino group.

**4.** The reagent for measuring skin sensitization according to claim 1, wherein the organic compound is represented by Formula (3), Formula (4), or Formula (5),

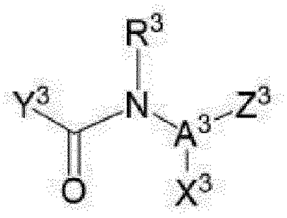

(3)

in the formula,

$A^3$ represents a trivalent hydrocarbon group having 1 or 2 carbon atoms,
$R^3$ represents a hydrogen atom, an alkyl group having 1 to 10 carbon atoms, or a cycloalkyl group having 3 to 10 carbon atoms, each of which may contain -O-, -C(O)-, -OC(O)-, $-NJ^{31}-CO-$, $-CO-NJ^{31}-$, or -NH-CO-NH- in a molecular chain, $J^{31}$ represents a hydrogen atom or an alkyl group having 1 to 3 carbon atoms, and the alkyl group or the cycloalkyl group may have a substituent selected from a cycloalkyl group having 3 to 6 carbon atoms, an amino group, a cyano group, a mercapto group, a hydroxyl group, a carboxyl group, a phenyl group, a hydroxyphenyl group, a pyridyl group, a naphthyl group, a thienyl group, or a furyl group,
$X^3$ represents an alkyl group having one or more mercapto groups and having 1 to 10 carbon atoms, a cycloalkyl group having one or more mercapto groups and having 3 to 10 carbon atoms, an arylalkyl group having one or more mercapto groups and having 7 to 12 carbon atoms, a heteroalkylalkyl group having one or more mercapto groups and having 3 to 10 carbon atoms, or a mercapto group, each of which may contain -O-, -C(O)-, -OC(O)-, $-NJ^{32}-CO-$, $-CO-NJ^{32}-$, or -NH-CO-NH- in a molecular chain, $J^{32}$ represents a hydrogen atom or an alkyl group having 1 to 3 carbon atoms, and the alkyl group or the cycloalkyl group may have a substituent selected from a cycloalkyl group having 3 to 6 carbon atoms, an amino group, a cyano group, a hydroxyl group, a carboxyl group, a phenyl group, a hydroxyphenyl group, a pyridyl group, a naphthyl group, a thienyl group, or a furyl group,
$Y^3$ represents a group having 6 to 20 carbon atoms and containing a structure having an absorption spectrum in an ultraviolet, visible, or near-infrared region, and
$Z^3$ represents $-CO-NR^{31}NR^{32}R^{33}$, where $R^{31}$, $R^{32}$, and $R^{33}$ each independently represent a hydrogen atom or an alkyl group having 1 to 10 carbon atoms,

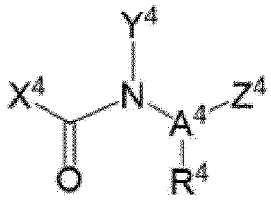

(4)

in the formula,

$A^4$ represents a trivalent hydrocarbon group having 1 or 2 carbon atoms,
$R^4$ represents a hydrogen atom, an alkyl group having 1 to 10 carbon atoms, or a cycloalkyl group having 3 to 10 carbon atoms, each of which may contain -O-, -C(O)-, -OC(O)-, $-NJ^{41}-CO-$, $-CO-NJ^{41}-$, or -NH-CO-NH- in a molecular chain, $J^{41}$ represents a hydrogen atom or an alkyl group having 1 to 3 carbon atoms, and the alkyl group or the cycloalkyl group may have a substituent selected from a cycloalkyl group having 3 to 6 carbon atoms, an amino group, a cyano group, a mercapto group, a hydroxyl group, a carboxyl group, a phenyl group, a hydroxyphenyl group, a pyridyl group, a naphthyl group, a thienyl group, or a furyl group,
$X^4$ represents an alkyl group having one or more mercapto groups and having 1 to 10 carbon atoms, a cycloalkyl group having one or more mercapto groups and having 3 to 10 carbon atoms, an arylalkyl group having one or more mercapto groups and having 7 to 12 carbon atoms, a heteroalkylalkyl group having one or more mercapto groups and having 3 to 10 carbon atoms, or a mercapto group, each of which may contain -O-, -C(O)-, -OC(O)-, $-NJ^{42}-CO-$, $-CO-NJ^{42}-$, or -NH-CO-NH- in a molecular chain, $J^{42}$ represents a hydrogen atom or an alkyl group having 1 to 3 carbon atoms, and the alkyl group or the cycloalkyl group may have a substituent selected from a cycloalkyl group having 3 to 6 carbon atoms, an amino group, a cyano group, a hydroxyl group, a carboxyl group, a phenyl group, a hydroxyphenyl group, a pyridyl group, a naphthyl group, a thienyl group, or a furyl group,
$Y^4$ represents a group having 6 to 20 carbon atoms and containing a structure having an absorption spectrum in an ultraviolet, visible, or near-infrared region, and

$Z^4$ represents -CO-$NR^{41}NR^{42}R^{43}$, where $R^{41}$, $R^{42}$, and $R^{43}$ each independently represent a hydrogen atom or an alkyl group having 1 to 10 carbon atoms,

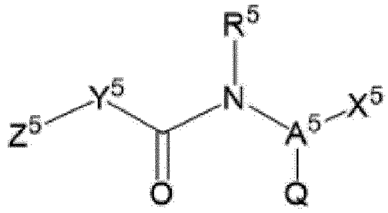

(5)

in the formula,

$A^5$ represents a trivalent hydrocarbon group having 1 or 2 carbon atoms,
$R^5$ represents a hydrogen atom, an alkyl group having 1 to 10 carbon atoms, or a cycloalkyl group having 3 to 10 carbon atoms, each of which may contain -O-, -C(O)-, -OC(O)-, -$NJ^{51}$-CO-, -CO-$NJ^{51}$-, or -NH-CO-NH- in a molecular chain, $J^{51}$ represents a hydrogen atom or an alkyl group having 1 to 3 carbon atoms, and the alkyl group or the cycloalkyl group may have a substituent selected from a cycloalkyl group having 3 to 6 carbon atoms, an amino group, a cyano group, a mercapto group, a hydroxyl group, a carboxyl group, a phenyl group, a hydroxyphenyl group, a pyridyl group, a naphthyl group, a thienyl group, or a furyl group,
$X^5$ represents an alkyl group having one or more mercapto groups and having 1 to 10 carbon atoms, a cycloalkyl group having one or more mercapto groups and having 3 to 10 carbon atoms, an arylalkyl group having one or more mercapto groups and having 7 to 12 carbon atoms, a heteroalkylalkyl group having one or more mercapto groups and having 3 to 10 carbon atoms, or a mercapto group, each of which may contain -O-, -C(O)-, -OC(O)-, -$NJ^{52}$-CO-, -CO-$NJ^{52}$-, or -NH-CO-NH- in a molecular chain, $J^{52}$ represents a hydrogen atom or an alkyl group having 1 to 3 carbon atoms, and the alkyl group or the cycloalkyl group may have a substituent selected from a cycloalkyl group having 3 to 6 carbon atoms, an amino group, a cyano group, a hydroxyl group, a carboxyl group, a phenyl group, a hydroxyphenyl group, a pyridyl group, a naphthyl group, a thienyl group, or a furyl group,
$Y^5$ represents a group having 6 to 20 carbon atoms and containing a structure having an absorption spectrum in an ultraviolet, visible, or near-infrared region,
$Z^5$ represents -CO-$NR^{51}NR^{52}R^{53}$, where $R^{51}$, $R^{52}$, and $R^{53}$ each independently represent a hydrogen atom or an alkyl group having 1 to 10 carbon atoms, and
Q represents a hydrogen atom, a carboxyl group, a hydroxyl group, or a primary amide structure, or represents -CO-$NR^{5a}NR^{5b}R^{5c}$, where $R^{5a}$, $R^{5b}$, and $R^{5c}$ each independently represent a hydrogen atom or an alkyl group having 1 to 10 carbon atoms.

**5.** The reagent for measuring skin sensitization according to claim 1, wherein the organic compound is represented by Formula (6),

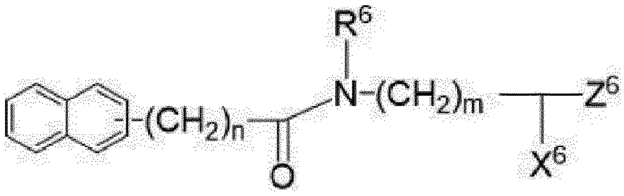

(6)

in the formula,

$R^6$ represents a hydrogen atom, an alkyl group having 1 to 10 carbon atoms, or a cycloalkyl group having 3 to 10 carbon atoms, each of which may contain -O-, -C(O)-, -OC(O)-, -$NJ^{61}$-CO-, -CO-$NJ^{61}$-, or -NH-CO-NH- in a molecular chain, $J^{61}$ represents a hydrogen atom or an alkyl group having 1 to 3 carbon atoms, and the alkyl group or the cycloalkyl group may have a substituent selected from a cycloalkyl group having 3 to 6 carbon atoms, an amino group, a cyano group, a mercapto group, a hydroxyl group, a carboxyl group, a phenyl group, a hydroxyphenyl group, a pyridyl group, a naphthyl group, a thienyl group, or a furyl group,
$X^6$ represents an alkyl group having one or more mercapto groups and having 1 to 10 carbon atoms, a cycloalkyl

group having one or more mercapto groups and having 3 to 10 carbon atoms, an arylalkyl group having one or more mercapto groups and having 7 to 12 carbon atoms, a heteroalkylalkyl group having one or more mercapto groups and having 3 to 10 carbon atoms, or a mercapto group, each of which may contain -O-, -C(O)-, -OC(O)-, -$NJ^{62}$-CO-, -CO-$NJ^{62}$-, or -NH-CO-NH- in a molecular chain, $J^{62}$ represents a hydrogen atom or an alkyl group having 1 to 3 carbon atoms, and the alkyl group or the cycloalkyl group may have a substituent selected from a cycloalkyl group having 3 to 6 carbon atoms, an amino group, a cyano group, a hydroxyl group, a carboxyl group, a phenyl group, a hydroxyphenyl group, a pyridyl group, a naphthyl group, a thienyl group, or a furyl group,
$Z^6$ represents -CO-$NR^{61}NR^{62}R^{63}$, where $R^{61}$, $R^{62}$, and $R^{63}$ each independently represent a hydrogen atom or an alkyl group having 1 to 10 carbon atoms,
n represents 0 or 1, and
m represents 0 or 1.

**6.** The reagent for measuring skin sensitization according to claim 1, wherein the organic compound is represented by Formula (7),

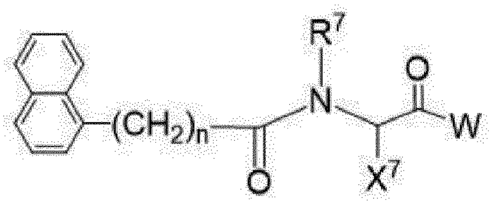
(7)

in the formula,

$R^7$ represents a hydrogen atom, an alkyl group having 1 to 10 carbon atoms, or a cycloalkyl group having 3 to 10 carbon atoms, each of which may contain -O-, -C(O)-, -OC(O)-, -$NJ^{71}$-CO-, -CO-$NJ^{71}$-, or -NH-CO-NH- in a molecular chain, $J^{71}$ represents a hydrogen atom or an alkyl group having 1 to 3 carbon atoms, and the alkyl group or the cycloalkyl group may have a substituent selected from a cycloalkyl group having 3 to 6 carbon atoms, an amino group, a cyano group, a mercapto group, a hydroxyl group, a carboxyl group, a phenyl group, a hydroxyphenyl group, a pyridyl group, a naphthyl group, a thienyl group, or a furyl group,
$X^7$ represents an alkyl group having one or more mercapto groups and having 1 to 10 carbon atoms, a cycloalkyl group having one or more mercapto groups and having 3 to 10 carbon atoms, an arylalkyl group having one or more mercapto groups and having 7 to 12 carbon atoms, a heteroalkylalkyl group having one or more mercapto groups and having 3 to 10 carbon atoms, or a mercapto group, each of which may contain -O-, -C(O)-, -OC(O)-, -$NJ^{72}$-CO-, -CO-$NJ^{72}$-, or -NH-CO-NH- in a molecular chain, $J^{72}$ represents a hydrogen atom or an alkyl group having 1 to 3 carbon atoms, and the alkyl group or the cycloalkyl group may have a substituent selected from a cycloalkyl group having 3 to 6 carbon atoms, an amino group, a cyano group, a hydroxyl group, a carboxyl group, a phenyl group, a hydroxyphenyl group, a pyridyl group, a naphthyl group, a thienyl group, or a furyl group,
W represents $NR^{71}$-$NR^{72}R^{73}$, where $R^{71}$, $R^{72}$, and $R^{73}$ represent a hydrogen atom or an alkyl group having 1 to 3 carbon atoms, and
n represents 0 or 1.

**7.** The reagent for measuring skin sensitization according to any one of claims 2 to 4, wherein $Y^1$, $Y^2$, $Y^3$, $Y^4$, $Y^5$, and $Y^{10}$ are groups that emit fluorescence.

**8.** A compound represented by Formula (3), Formula (4), or Formula (5),

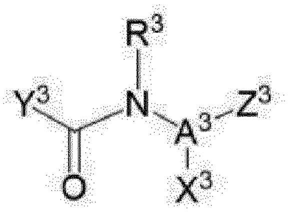
(3)

in the formula,

$A^3$ represents a trivalent hydrocarbon group having 1 or 2 carbon atoms,
$R^3$ represents a hydrogen atom, an alkyl group having 1 to 10 carbon atoms, or a cycloalkyl group having 3 to 10 carbon atoms, each of which may contain -O-, -C(O)-, -OC(O)-, $-NJ^{31}-CO-$, $-CO-NJ^{31}-$, or -NH-CO-NH- in a molecular chain, $J^{31}$ represents a hydrogen atom or an alkyl group having 1 to 3 carbon atoms, and the alkyl group or the cycloalkyl group may have a substituent selected from a cycloalkyl group having 3 to 6 carbon atoms, an amino group, a cyano group, a mercapto group, a hydroxyl group, a carboxyl group, a phenyl group, a hydroxyphenyl group, a pyridyl group, a naphthyl group, a thienyl group, or a furyl group,
$X^3$ represents an alkyl group having one or more mercapto groups and having 1 to 10 carbon atoms, a cycloalkyl group having one or more mercapto groups and having 3 to 10 carbon atoms, an arylalkyl group having one or more mercapto groups and having 7 to 12 carbon atoms, a heteroalkylalkyl group having one or more mercapto groups and having 3 to 10 carbon atoms, or a mercapto group, each of which may contain -O-, -C(O)-, -OC(O)-, $-NJ^{32}-CO-$, $-CO-NJ^{32}-$, or -NH-CO-NH- in a molecular chain, $J^{32}$ represents a hydrogen atom or an alkyl group having 1 to 3 carbon atoms, and the alkyl group or the cycloalkyl group may have a substituent selected from a cycloalkyl group having 3 to 6 carbon atoms, an amino group, a cyano group, a hydroxyl group, a carboxyl group, a phenyl group, a hydroxyphenyl group, a pyridyl group, a naphthyl group, a thienyl group, or a furyl group,
$Y^3$ represents a group having 6 to 20 carbon atoms and containing a structure having an absorption spectrum in an ultraviolet, visible, or near-infrared region, and
$Z^3$ represents $-CO-NR^{31}NR^{32}R^{33}$, where $R^{31}$, $R^{32}$, and $R^{33}$ each independently represent a hydrogen atom or an alkyl group having 1 to 10 carbon atoms,

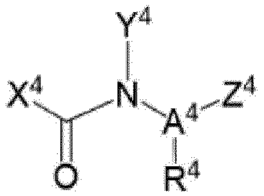

(4)

in the formula,

$A^4$ represents a trivalent hydrocarbon group having 1 or 2 carbon atoms,
$R^4$ represents a hydrogen atom, an alkyl group having 1 to 10 carbon atoms, or a cycloalkyl group having 3 to 10 carbon atoms, each of which may contain -O-, -C(O)-, -OC(O)-, $-NJ^{41}-CO-$, $-CO-NJ^{41}-$, or -NH-CO-NH- in a molecular chain, $J^{41}$ represents a hydrogen atom or an alkyl group having 1 to 3 carbon atoms, and the alkyl group or the cycloalkyl group may have a substituent selected from a cycloalkyl group having 3 to 6 carbon atoms, an amino group, a cyano group, a mercapto group, a hydroxyl group, a carboxyl group, a phenyl group, a hydroxyphenyl group, a pyridyl group, a naphthyl group, a thienyl group, or a furyl group,
$X^4$ represents an alkyl group having one or more mercapto groups and having 1 to 10 carbon atoms, a cycloalkyl group having one or more mercapto groups and having 3 to 10 carbon atoms, an arylalkyl group having one or more mercapto groups and having 7 to 12 carbon atoms, a heteroalkylalkyl group having one or more mercapto groups and having 3 to 10 carbon atoms, or a mercapto group, each of which may contain -O-, -C(O)-, -OC(O)-, $-NJ^{42}-CO-$, $-CO-NJ^{42}-$, or -NH-CO-NH- in a molecular chain, $J^{42}$ represents a hydrogen atom or an alkyl group having 1 to 3 carbon atoms, and the alkyl group or the cycloalkyl group may have a substituent selected from a cycloalkyl group having 3 to 6 carbon atoms, an amino group, a cyano group, a hydroxyl group, a carboxyl group, a phenyl group, a hydroxyphenyl group, a pyridyl group, a naphthyl group, a thienyl group, or a furyl group,
$Y^4$ represents a group having 6 to 20 carbon atoms and containing a structure having an absorption spectrum in an ultraviolet, visible, or near-infrared region, and
$Z^4$ represents $-CO-NR^{41}NR^{42}R^{43}$, where $R^{41}$, $R^{42}$, and $R^{43}$ each independently represent a hydrogen atom or an alkyl group having 1 to 10 carbon atoms,

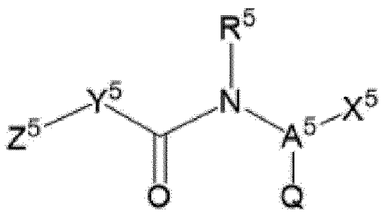

(5)

in the formula,

$A^5$ represents a trivalent hydrocarbon group having 1 or 2 carbon atoms,
$R^5$ represents a hydrogen atom, an alkyl group having 1 to 10 carbon atoms, or a cycloalkyl group having 3 to 10 carbon atoms, each of which may contain -O-, -C(O)-, -OC(O)-, $-NJ^{51}-CO-$, $-CO-NJ^{51}-$, or -NH-CO-NH- in a molecular chain, $J^{51}$ represents a hydrogen atom or an alkyl group having 1 to 3 carbon atoms, and the alkyl group or the cycloalkyl group may have a substituent selected from a cycloalkyl group having 3 to 6 carbon atoms, an amino group, a cyano group, a mercapto group, a hydroxyl group, a carboxyl group, a phenyl group, a hydroxyphenyl group, a pyridyl group, a naphthyl group, a thienyl group, or a furyl group,
$X^5$ represents an alkyl group having one or more mercapto groups and having 1 to 10 carbon atoms, a cycloalkyl group having one or more mercapto groups and having 3 to 10 carbon atoms, an arylalkyl group having one or more mercapto groups and having 7 to 12 carbon atoms, a heteroalkylalkyl group having one or more mercapto groups and having 3 to 10 carbon atoms, or a mercapto group, each of which may contain -O-, -C(O)-, -OC(O)-, $-NJ^{52}-CO-$, $-CO-NJ^{52}-$, or -NH-CO-NH- in a molecular chain, $J^{52}$ represents a hydrogen atom or an alkyl group having 1 to 3 carbon atoms, and the alkyl group or the cycloalkyl group may have a substituent selected from a cycloalkyl group having 3 to 6 carbon atoms, an amino group, a cyano group, a hydroxyl group, a carboxyl group, a phenyl group, a hydroxyphenyl group, a pyridyl group, a naphthyl group, a thienyl group, or a furyl group,
$Y^5$ represents a group having 6 to 20 carbon atoms and containing a structure having an absorption spectrum in an ultraviolet, visible, or near-infrared region,
$Z^5$ represents $-CO-NR^{51}NR^{52}R^{53}$, where $R^{51}$, $R^{52}$, and $R^{53}$ each independently represent a hydrogen atom or an alkyl group having 1 to 10 carbon atoms, and
Q represents a hydrogen atom, a carboxyl group, a hydroxyl group, or a primary amide structure, or represents $-CO-NR^{5a}NR^{5b}R^{5c}$, where $R^{5a}$, $R^{5b}$, and $R^{5c}$ each independently represent a hydrogen atom or an alkyl group having 1 to 10 carbon atoms.

**9.** A compound represented by Formula (6),

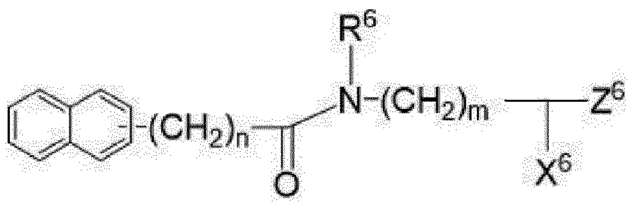

(6)

in the formula,

$R^6$ represents a hydrogen atom, an alkyl group having 1 to 10 carbon atoms, or a cycloalkyl group having 3 to 10 carbon atoms, each of which may contain -O-, -C(O)-, -OC(O)-, $-NJ^{61}-CO-$, $-CO-NJ^{61}-$, or -NH-CO-NH- in a molecular chain, $J^{61}$ represents a hydrogen atom or an alkyl group having 1 to 3 carbon atoms, and the alkyl group or the cycloalkyl group may have a substituent selected from a cycloalkyl group having 3 to 6 carbon atoms, an amino group, a cyano group, a mercapto group, a hydroxyl group, a carboxyl group, a phenyl group, a hydroxyphenyl group, a pyridyl group, a naphthyl group, a thienyl group, or a furyl group,
$X^6$ represents an alkyl group having one or more mercapto groups and having 1 to 10 carbon atoms, a cycloalkyl group having one or more mercapto groups and having 3 to 10 carbon atoms, an arylalkyl group having one or more mercapto groups and having 7 to 12 carbon atoms, a heteroalkylalkyl group having one or more mercapto groups and having 3 to 10 carbon atoms, or a mercapto group, each of which may contain -O-, -C(O)-, -OC(O)-, $-NJ^{62}-CO-$, $-CO-NJ^{62}-$, or -NH-CO-NH- in a molecular chain, $J^{62}$ represents a hydrogen atom or an alkyl group having 1 to 3 carbon atoms, and the alkyl group or the cycloalkyl group may have a substituent selected from a cycloalkyl group having 3 to 6 carbon atoms, an amino group, a cyano group, a hydroxyl group, a carboxyl group, a phenyl group, a hydroxyphenyl group, a pyridyl group, a naphthyl group, a thienyl group, or a furyl group,
$Z^6$ represents $-CO-NR^{61}NR^{62}R^{63}$, where $R^{61}$, $R^{62}$, and $R^{63}$ each independently represent a hydrogen atom or an alkyl group having 1 to 10 carbon atoms,
n represents 0 or 1, and
m represents 0 or 1.

**10.** A compound represented by Formula (7),

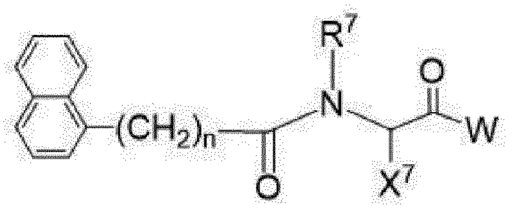 (7)

in the formula,

$R^7$ represents a hydrogen atom, an alkyl group having 1 to 10 carbon atoms, or a cycloalkyl group having 3 to 10 carbon atoms, each of which may contain -O-, -C(O)-, -OC(O)-, $-NJ^{71}-CO-$, $-CO-NJ^{71}-$, or -NH-CO-NH- in a molecular chain, $J^{71}$ represents a hydrogen atom or an alkyl group having 1 to 3 carbon atoms, and the alkyl group or the cycloalkyl group may have a substituent selected from a cycloalkyl group having 3 to 6 carbon atoms, an amino group, a cyano group, a mercapto group, a hydroxyl group, a carboxyl group, a phenyl group, a hydroxyphenyl group, a pyridyl group, a naphthyl group, a thienyl group, or a furyl group,
$X^7$ represents an alkyl group having one or more mercapto groups and having 1 to 10 carbon atoms, a cycloalkyl group having one or more mercapto groups and having 3 to 10 carbon atoms, an arylalkyl group having one or more mercapto groups and having 7 to 12 carbon atoms, a heteroalkylalkyl group having one or more mercapto groups and having 3 to 10 carbon atoms, or a mercapto group, each of which may contain -O-, -C(O)-, -OC(O)-, $-NJ^{72}-CO-$, $-CO-NJ^{72}-$, or -NH-CO-NH- in a molecular chain, $J^{72}$ represents a hydrogen atom or an alkyl group having 1 to 3 carbon atoms, and the alkyl group or the cycloalkyl group may have a substituent selected from a cycloalkyl group having 3 to 6 carbon atoms, an amino group, a cyano group, a hydroxyl group, a carboxyl group, a phenyl group, a hydroxyphenyl group, a pyridyl group, a naphthyl group, a thienyl group, or a furyl group,
W represents $NR^{71}-NR^{72}R^{73}$, where $R^{71}$, $R^{72}$, and $R^{73}$ represent a hydrogen atom or an alkyl group having 1 to 3 carbon atoms, and
n represents 0 or 1.

**11.** A method for measuring skin sensitization comprising:

(1) reacting the reagent for measuring skin sensitization according to any one of claims 1 to 7 with a test substance; and
(2) detecting an amount of the reagent for measuring skin sensitization after the reaction or an amount of a product of the reaction by optical measurement.

**12.** The method for measuring skin sensitization according to claim 11,
wherein the test substance is at least one of a fragrance, an essential oil, a polymer compound, a pharmaceutical, an agricultural chemical, a food, a chemical product, or a plant extract consisting of a natural product-derived component.

**13.** The method for measuring skin sensitization according to claim 11 or 12, further comprising:

subjecting a reaction product obtained in the step of reacting the reagent for measuring skin sensitization with the test substance to chromatography.

**14.** The method for measuring skin sensitization according to any one of claims 11 to 13,
wherein the optical measurement is a measurement using a fluorescence detector, an excitation wavelength is 200 to 600 nm, and a fluorescence wavelength is 200 to 800 nm.

FIG. 1

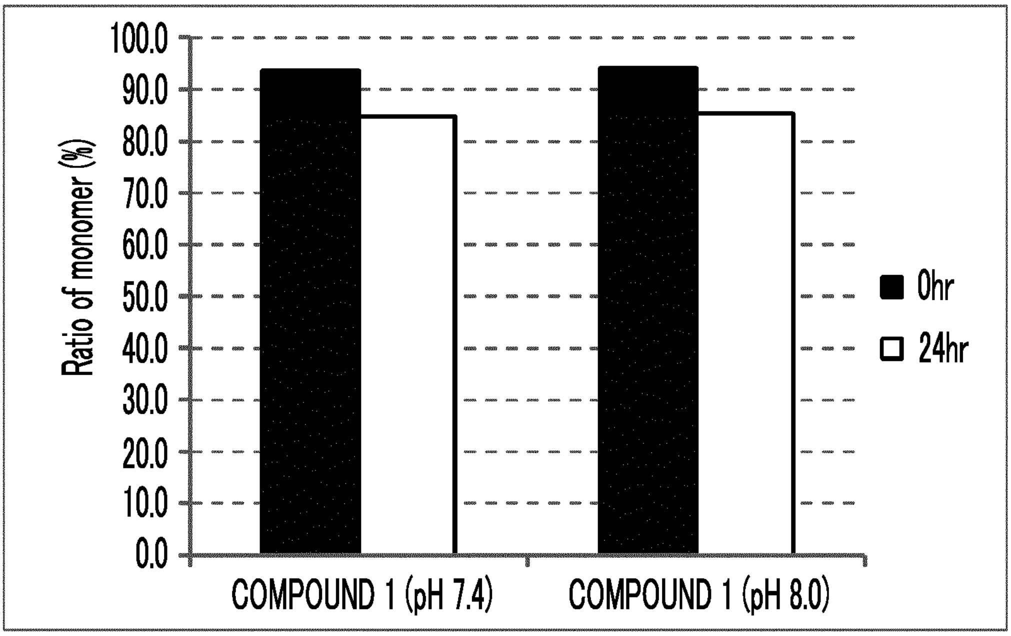
Ratio of monomer (%)
100.0
90.0
80.0
70.0
60.0
50.0
40.0
30.0
20.0
10.0
0.0
COMPOUND 1 (pH 7.4)
COMPOUND 1 (pH 8.0)
0hr
24hr

FIG. 2

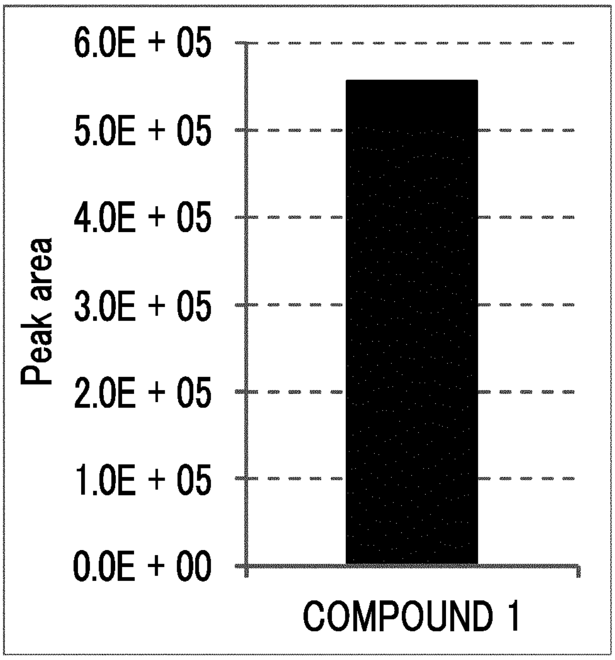
Peak area
6.0E + 05
5.0E + 05
4.0E + 05
3.0E + 05
2.0E + 05
1.0E + 05
0.0E + 00
COMPOUND 1

FIG. 3

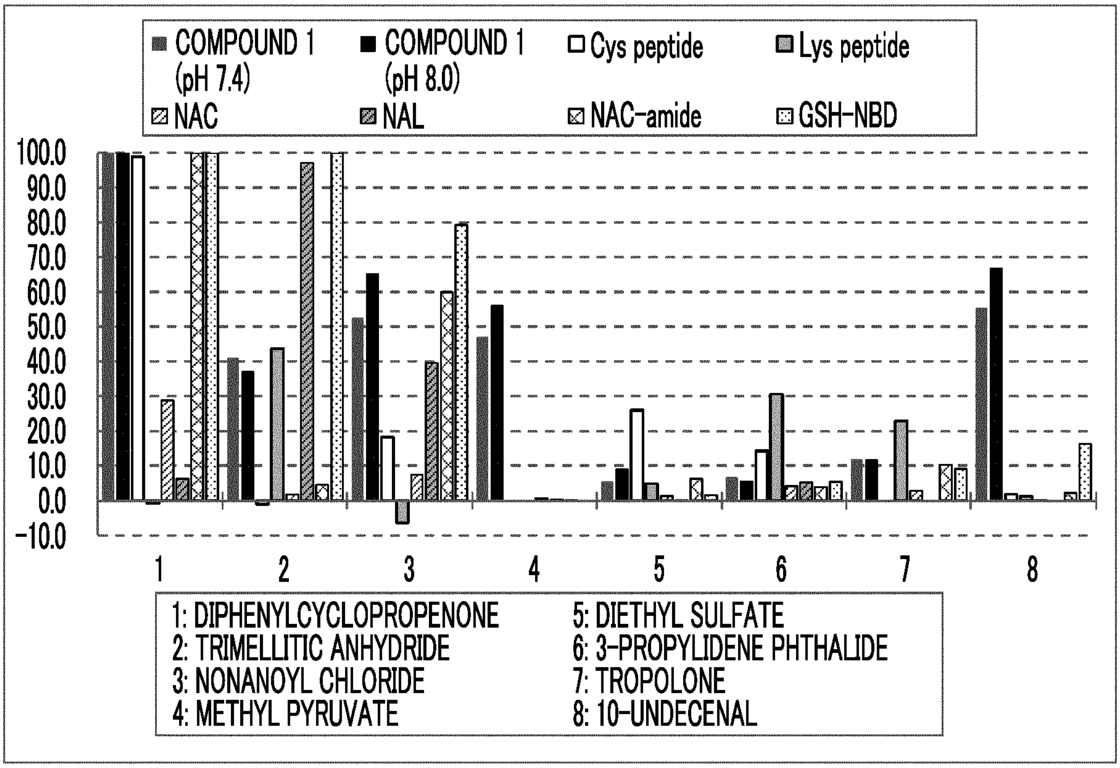
COMPOUND 1 (pH 7.4)
COMPOUND 1 (pH 8.0)
Cys peptide
Lys peptide
NAC
NAL
NAC-amide
GSH-NBD
100.0
90.0
80.0
70.0
60.0
50.0
40.0
30.0
20.0
10.0
0.0
-10.0
1
2
3
4
5
6
7
8
1: DIPHENYLCYCLOPROPENONE
2: TRIMELLITIC ANHYDRIDE
3: NONANOYL CHLORIDE
4: METHYL PYRUVATE
5: DIETHYL SULFATE
6: 3-PROPYLIDENE PHTHALIDE
7: TROPOLONE
8: 10-UNDECENAL

FIG. 4

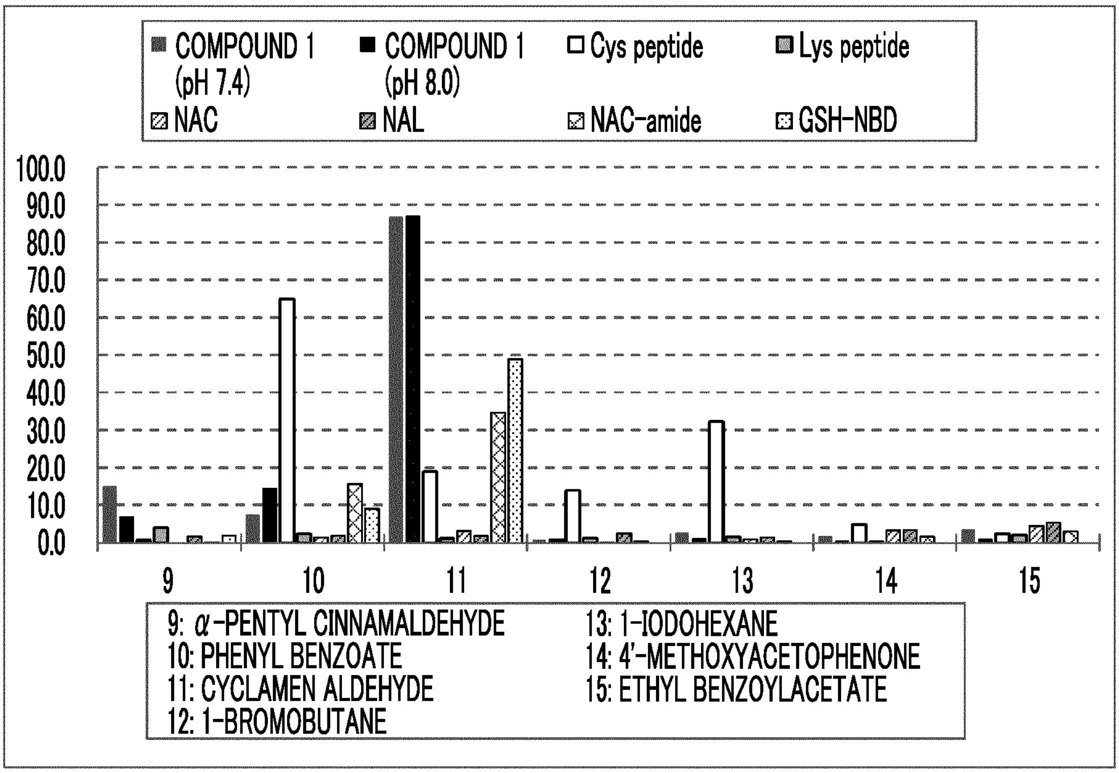
COMPOUND 1 (pH 7.4)
COMPOUND 1 (pH 8.0)
Cys peptide
Lys peptide
NAC
NAL
NAC-amide
GSH-NBD
100.0
90.0
80.0
70.0
60.0
50.0
40.0
30.0
20.0
10.0
0.0
9
10
11
12
13
14
15
9: α-PENTYL CINNAMALDEHYDE
10: PHENYL BENZOATE
11: CYCLAMEN ALDEHYDE
12: 1-BROMOBUTANE
13: 1-IODOHEXANE
14: 4'-METHOXYACETOPHENONE
15: ETHYL BENZOYLACETATE

**INTERNATIONAL SEARCH REPORT**

International application No. **PCT/JP2021/038929**

**A. CLASSIFICATION OF SUBJECT MATTER**

***C07C 321/04***(2006.01)i; ***C07C 321/26***(2006.01)i; ***C07D 333/38***(2006.01)i; ***G01N 31/00***(2006.01)i; ***C07D 213/72***(2006.01)i; ***G01N 33/15***(2006.01)i; ***G01N 21/17***(2006.01)i; ***G01N 21/31***(2006.01)i; ***G01N 21/64***(2006.01)i; ***G01N 30/06***(2006.01)i
FI: G01N33/15 Z; C07C321/26; G01N31/00 V; G01N30/06 E; C07D213/72; C07D333/38; G01N21/64 F; G01N21/17 D; G01N21/31; C07C321/04 CSP

According to International Patent Classification (IPC) or to both national classification and IPC

**B. FIELDS SEARCHED**

Minimum documentation searched (classification system followed by classification symbols)

C07C321/04; C07C321/26; C07D333/38; G01N31/00; C07D213/72; G01N33/15; G01N21/17; G01N21/31; G01N21/64; G01N30/06

Documentation searched other than minimum documentation to the extent that such documents are included in the fields searched

Published examined utility model applications of Japan 1922-1996
Published unexamined utility model applications of Japan 1971-2021
Registered utility model specifications of Japan 1996-2021
Published registered utility model applications of Japan 1994-2021

Electronic data base consulted during the international search (name of data base and, where practicable, search terms used)

JSTPlus/JMEDPlus/JST7580 (JDreamIII); CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| X | US 2014/0227793 A1 (CALIFORNIA INSTITUTE OF TECHNOLOGY) 14 August 2014 (2014-08-14)<br>paragraph [0115] | 8 |
| A | | 1-7, 9-14 |
| A | JP 57-45458 A (FUJI PHOTO FILM CO LTD) 15 March 1982 (1982-03-15)<br>publication gazette, p. 6, upper right column | 1-14 |
| A | WO 2020/045621 A1 (FUJIFILM CORP) 05 March 2020 (2020-03-05)<br>claims | 1-14 |
| A | JP 2009-222466 A (SUMITOMO CHEMICAL CO LTD) 01 October 2009 (2009-10-01)<br>claims | 1-14 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **15 December 2021** | **28 December 2021** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | <br>Telephone No. |

Form PCT/ISA/210 (second sheet) (January 2015)

INTERNATIONAL SEARCH REPORT

International application No.
PCT/JP2021/038929

C. DOCUMENTS CONSIDERED TO BE RELEVANT

| Category* | Citation of document, with indication, where appropriate, of the relevant passages | Relevant to claim No. |
|---|---|---|
| A | 須藤 英典 他, 皮膚感作性評価の現状と今後の展望, 住友化学, 2019, pp. 24-36, non-official translation (SUTO, Hidenori et al. Perspectives on the Current State of Evaluation of Skin Sensitization. SUMITOMO CHEMICAL CO LTD.) in particular, p. 29 | 1-14 |
| A | AKIMOTO, Miyuki et al. Oxidation of a cysteine-derived nucleophilic reagent by dimethyl sulfoxide in the amino acid derivative reactivity assay. Journal of Applied Toxicology. 12 February 2020, vol. 40, pp. 843-854 abstract | 1-14 |
| A | CHO, Sun-A et al. High-throughput screening (HTS)-based spectrophotometric direct peptide reactivity assay (Spectro-DPRA) to predict human skin sensitization potential. Toxicology Letters. 2019, vol. 314, pp. 27-36 abstract | 1-14 |

Form PCT/ISA/210 (second sheet) (January 2015)

INTERNATIONAL SEARCH REPORT

International application No.
PCT/JP2021/038929

**Box No. III Observations where unity of invention is lacking (Continuation of item 3 of first sheet)**

This International Searching Authority found multiple inventions in this international application, as follows:

Document 1: US 2014/0227793 A1 (CALIFORNIA INSTITUTE OF TECHNOLOGY) 14 August 2014 (2014-08-14), paragraph [0115]
Document 2: JP 57-45458 A (FUJI PHOTO FILM CO LTD) 15 March 1982 (1982-03-15), publication gazette, page 6, upper right column

(Invention 1) Claims 1-7 and 11-14

Claims 1-7 and 11-14 have the special technical feature of a “skin sensitization measuring reagent comprising, as a measurement principal agent, an organic compound which has a mercapto group and a hydrazide structure and has an absorption spectrum in an ultraviolet, visible, or near-infrared range”, and are thus classified as invention 1.

(Invention 2) Parts in claim 8, in which compound represented by formula (3) is selected, and claims 9-10

Claim 8 sets forth three independent inventions including a “compound represented by formula (3)”, a “compound represented by formula (4)”, and a “compound represented by formula (5)”. Moreover, the parts in claim 8, in which the compound represented by formula (3) is selected, share, with claim 1 classified as invention 1, the common technical feature of an “organic compound which has a mercapto group and a hydrazide structure and has an absorption spectrum in an ultraviolet, visible, or near-infrared range”. However, said technical feature does not make a contribution over the prior art in light of the disclosures of documents 1-2 and thus cannot be said to be a special technical feature. Further, there are no other same or corresponding special technical features between these inventions. The second structure from the bottom of the left column on page 17 of document 1 is a structure that has, from the right, a hydrazide group, a C1 trivalent hydrocarbon group substituted with a C1 alkyl group having a mercapto group, and an amide bond. Furthermore, the part on the left side of the amide bond is considered to have an absorption spectrum in an ultraviolet, visible, or near-infrared range, through the structure thereof. Therefore, document 1 discloses the compound represented by formula (3). Moreover, document 2 discloses the organic compound which has a mercapto group and a hydrazide structure and has an absorption spectrum in an ultraviolet, visible, or near-infrared range (compound examples H-18 and H-19).

In addition, the parts in claim 8, in which the compound represented by formula (3) is selected, are not dependent on claim 1. Moreover, the parts are not substantially identical to or similarly closely related to any of the claims classified as invention 1.

Accordingly, the parts in claim 8, in which the compound represented by formula (3) is selected, and claims 9-10 cannot be classified as invention 1.

(Invention 3) Parts in claim 8 in which compound represented by formula (4) is selected

The parts in claim 8, in which the compound represented by formula (4) is selected, share, with claim 1 classified as invention 1, the common technical feature of an “organic compound which has a mercapto group and a hydrazide structure and has an absorption spectrum in an ultraviolet, visible, or near-infrared range”. However, said technical feature does not make a contribution over the prior art in light of the disclosures of documents 1-2 and thus cannot be said to be a special technical feature. Further, there are no other same or corresponding special technical features between these inventions.

In addition, the compound represented by formula (3) and the compound represented by formula (4) share the common technical feature of a “compound having a ‘C1 or C2 trivalent hydrocarbon group’, a ‘mercapto group’, a ‘C6-C20 group including a structure having an absorption spectrum in an ultraviolet, visible, or near-infrared range’, and a ‘hydrazide group’”. However, said technical feature does not make a contribution over the prior art in light of the disclosure of document 1 and thus cannot be said to be a special technical feature. Further, there are no other same or corresponding special technical features between these inventions.

Furthermore, the parts in claim 8, in which the compound represented by formula (4) is selected, are not dependent on claim 1 or the parts in claim 8 in which the compound represented by formula (3) is selected. Moreover, the parts are not substantially identical to or similarly closely related to any of the claims classified as invention 1 or 2.

Accordingly, the parts in claim 8, in which the compound represented by formula (4) is selected, cannot be classified as invention 1 or 2.

(Invention 4) Parts in claim 8 in which compound represented by formula (5) is selected

The parts in claim 8, in which the compound represented by formula (5) is selected, share, with claim 1 classified as invention 1, the common technical feature of an “organic compound which has a mercapto group and a hydrazide structure and has an absorption spectrum in an ultraviolet, visible, or near-infrared range”. However, said technical

Form PCT/ISA/210 (continuation of first sheet) (January 2015)

**INTERNATIONAL SEARCH REPORT**

International application No.
**PCT/JP2021/038929**

**Box No. III Observations where unity of invention is lacking (Continuation of item 3 of first sheet)**

feature does not make a contribution over the prior art in light of the disclosures of documents 1-2 and thus cannot be said to be a special technical feature. Further, there are no other same or corresponding special technical features between these inventions.

In addition, the compound represented by formula (3) and the compound represented by formula (5) share the common technical feature of a "compound having a 'C1 or C2 trivalent hydrocarbon group', a 'mercapto group', a 'C6-C20 group including a structure having an absorption spectrum in an ultraviolet, visible, or near-infrared range', and a 'hydrazide group'". However, said technical feature does not make a contribution over the prior art in light of the disclosure of document 1 and thus cannot be said to be a special technical feature. Further, there are no other same or corresponding special technical features between these inventions.

Furthermore, the parts in claim 8, in which the compound represented by formula (5) is selected, are not dependent on claim 1 or the parts in claim 8 in which the compound represented by formula (3) is selected. Moreover, the parts are not substantially identical to or similarly closely related to any of the claims classified as any of invention 1-3.

Accordingly, the parts in claim 8, in which the compound represented by formula (5) is selected, cannot be classified as any of invention 1-3.

1. ☑ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☑ No protest accompanied the payment of additional search fees.

**INTERNATIONAL SEARCH REPORT**
**Information on patent family members**

International application No.
**PCT/JP2021/038929**

| Patent document cited in search report | Publication date (day/month/year) | Patent family member(s) | Publication date (day/month/year) |
|---|---|---|---|
| US 2014/0227793 A1 | 14 August 2014 | (Family: none) | |
| JP 57-45458 A | 15 March 1982 | US 4404289 A<br>column 8<br>EP 47472 A1 | |
| WO 2020/045621 A1 | 05 March 2020 | US 2021/0181109 A1<br>claims | |
| JP 2009-222466 A | 01 October 2009 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2011059102 A
- JP 2014037995 A
- JP 2009222466 A
- JP 2003014761 A
- JP 2008139275 A

### Non-patent literature cited in the description


- **GERBERICK, G. F. ; VASSALLO, J. D. ; BAILEY, R. E. ; CHANEY, J. G. ; MORRALL, S. W. ; LEPOIT-TEVIN, J. P.** Development of a peptide reactivity assay for screening contact allergens. *Toxicological Sciences,* 2004, vol. 81 (2), 332-343
- **GERBERICK, G. F. ; VASSALLO, J. D. ; FOERTSCH, L. M. ; PRICE, B. B. ; CHANEY, J. G. ; LEPOITTEVIN, J. P.** Quantification of chemical peptide reactivity for screening contact allergens: a classification tree model approach. *Toxicological Sciences,* 2007, vol. 97 (2), 417-427
- **DENNEHY M. K. ; RICHARDS K. A. M. ; WERNKE G. R. ; SHYR Y ; LIEBLER D. C.** Cytosolic and nuclear protein targets of thiol-reactive electrophiles. *Chemical Research in Toxicology,* 2006, vol. 19, 20-29
- **NATSCH A. ; GFELLER H.** LC-MS-based characterization of the peptide reactivity of chemicals to improve the in vitro prediction of the skin sensitization potential. *Toxicological Sciences,* 2008, vol. 106 (2), 464-478
- **WONG C. L. ; LAM A. L. ; SMITH M. T. ; GHASSABIAN S.** Evaluation of a High-Throughput Peptide Reactivity Format Assay for Assessment of the Skin Sensitization Potential of Chemicals. *Frontiers in Pharmacology,* 2016, vol. 14, 7 (53), 1-14
- **GERBERICK, G. F. ; VASSALLO, J. D. ; FOERTSCH, L. M. ; PRICE, B. B. ; CHANEY, J. G. ; LEPOITTEVIN, J. P.** Quantification of chemical peptide reactivity for screening contact allergens: a classification tree model approach. *Toxicological Sciences,* 2007, vol. 97 (2), 417-427
- **WONG C. L. ; LAM A. L. ; SMITH M. T. ; GHASSABIAN S.** Evaluation of a High-Throughput Peptide Reactivity Format Assay for Assessment of the Skin Sensitization Potential of Chemicals. *Frontiers in Pharmacology,* 2016, vol. 7 (53), 1-14
- *Journal of Medicinal Chemistry,* 1996, vol. 39 (7), 136
- *Bioorganic and Medicinal Chemistry,* 2008, vol. 16 (1), 65
- *Organic Letters,* 2013, vol. 15 (19), 5076
- *Tetrahedron Letters,* 2005, vol. 46 (43), 7443
- *Synlett,* 2010, (7), 1037